# EUROPÄISCHE PATENTSCHRIFT

(11) **EP 2 906 573 B1**
(45) Veröffentlichungstag und Bekanntmachung des Hinweises auf die Patenterteilung: **23.11.2016**
(21) Anmeldenummer: 13773208.7
(22) Anmeldetag: 27.09.2013
(51) Int. Cl.: C07F 9/6574, B01J 31/02, B01J 31/18, C07C 45/50

(54) **GEMISCH VON BISPHOSPHITEN UND DESSEN VERWENDUNG ALS KATALYSATORGEMISCH IN DER HYDROFORMYLIERUNG**
MIXTURE OF BIPHOSPHITES AND THEIR USE AS CATALYST MIXTURE FOR HYDROFORMYLATION
MÉLANGE DE BIPHOSPHITES ET LEUR UTILISATION COMME MÉLANGE CATALYTIQUE POUR L'HYDROFORMYLATION

(30) Priorität: 12.10.2012 DE 102012218627; 12.10.2012 DE 102012218625; 12.10.2012 DE 102012218629; 12.10.2012 DE 102012218630
(43) Veröffentlichungstag der Anmeldung: 19.08.2015
(73) Patentinhaber: Evonik Degussa GmbH, 45128 Essen (DE)
(72) Erfinder: CHRISTIANSEN, Andrea, 18119 Rostock (DE); FRANKE, Robert, 45772 Marl (DE); FRIDAG, Dirk, 45721 Haltern am See (DE); HESS, Dieter, 45770 Marl (DE); DYBALLA, Katrin Marie, 45657 Recklinghausen (DE); HANNEBAUER, Bernd, 63165 Mühlheim (DE)
(86) Internationale Anmeldenummer: PCT/EP2013/070226
(87) Internationale Veröffentlichungsnummer: WO 2014/056736

(56) Entgegenhaltungen:
- EP-B1- 1 294 731
- DE-A1-102008 002 187
- US-A- 4 769 498

## Beschreibung

Die Erfindung betrifft ein Gemisch von Bisphosphiten, ein Verfahren zu dessen Herstellung, sowie deren Umsetzung mit Metallen zu Gemischen enthaltenden Komplexverbindungen aus den konstitutionsisomeren Bisphosphiten und dem Metall, sowie deren Verwendung als katalytisch aktive Zusammensetzung in Hydroformylierungsreaktionen, wie auch die Hydroformylierungsreaktion selbst.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigem Phosphor P^{III}. Eine gute Übersicht über den Stand der Hydroformylierung von Olefinen findet sich in B. CORNILS, W. A. HERRMANN, "Applied Homogeneous Catalysis with Organometallic Compounds", Vol. 1 & 2, VCH, Weinheim, New York, 1996 bzw. R. Franke, D. Selent, A. Börner, "Applied Hydroformylation", Chem. Rev., 2012, DOI:10.1021/cr3001803.

Jede katalytisch aktive Zusammensetzung hat ihre spezifischen Vorzüge. Je nach Einsatzstoff und Zielprodukt kommen daher unterschiedliche katalytisch aktive Zusammensetzungen zum Einsatz.

Die Patente US 4 694 109 und US 4 879 416 beschreiben Bisphosphinliganden und ihren Einsatz in der Hydroformylierung von Olefinen bei niedrigen Synthesegasdrücken. Besonders bei der Hydroformylierung von Propen werden mit Liganden dieses Typs hohe Aktivitäten und hohe n/i-Selektivitäten (n/i = das Verhältnis von linearem Aldehyd (=n) zu verzweigtem (=iso) Aldehyd) erreicht. In WO 95/30680 werden zweizähnige Phosphinliganden und ihr Einsatz in der Katalyse, unter anderem auch in Hydroformylierungsreaktionen, offen gelegt. Ferrocenverbrückte Bisphosphine werden beispielsweise in den Patentschriften US 4 169 861, US 4 201 714 und US 4 193 943 als Liganden für Hydroformylierungen beschrieben.

Der Nachteil von zwei- und mehrzähnigen Phosphinliganden ist ein relativ hoher Aufwand, der zu ihrer Darstellung notwendig ist. Daher ist es oftmals nicht rentabel, solche Systeme in technischen Prozessen einzusetzen. Hinzu kommt eine vergleichsweise geringe Aktivität, die durch hohe Verweilzeiten reaktionstechnisch kompensiert werden muss. Dies wiederum führt zu unerwünschten Nebenreaktionen der Produkte.

Rhodium-Monophosphit-Komplexe in katalytisch aktiven Zusammensetzungen sind geeignet für die Hydroformylierung von verzweigten Olefinen mit innenständigen Doppelbindungen, jedoch ist die Selektivität für endständig oxierte Verbindungen gering. Aus EP 0 155 508 ist die Verwendung von bisarylensubstituierten Monophosphiten bei der rhodiumkatalysierten Hydroformylierung von sterisch gehinderten Olefinen, z. B. Isobuten, bekannt.

Katalytisch aktive Zusammensetzungen auf Basis von Rhodium-Bisphosphit-Komplexen sind geeignet für die Hydroformylierung von linearen Olefinen mit end- und innenständigen Doppelbindungen, wobei überwiegend endständig hydroformylierte Produkte entstehen. Dagegen werden verzweigte Olefine mit innenständigen Doppelbindungen nur im geringen Masse umgesetzt. Diese Phosphite ergeben bei ihrer Koordination an ein Übergangsmetallzentrum Katalysatoren von gesteigerter Aktivität, doch ist das Standzeitverhalten dieser katalytisch aktiven Zusammensetzungen, unter anderem wegen der Hydrolyseempfindlichkeit der Phosphitliganden, unbefriedigend. Durch den Einsatz von substituierten Bisaryldiolen als Bausteine für die Phosphitliganden, wie in EP 0 214 622 oder EP 0 472 071 beschrieben, konnten erhebliche Verbesserungen erreicht werden.

Der Literatur zufolge sind die katalytisch aktiven Zusammensetzungen dieser Liganden auf Basis von Rhodium äußerst aktiv in der Hydroformylierung von α-Olefinen. In den Patenten US 4 668 651, US 4 748 261 und US 4 885 401 werden Polyphosphitliganden beschrieben, mit denen α-Olefine, aber auch 2-Buten mit hoher n/i-Selektivität zu den terminal oxierten Produkten umgesetzt werden können. Zweizähnige Liganden dieses Typs wurden auch zur Hydroformylierung von Butadien eingesetzt (US 5 312 996).

Die in EP 1 294 731 offenbarten Bisphosphite weisen bei der Hydroformylierung von n-Octengemischen Olefinumsätze bis zu 98 % auf. Jedoch ist die ebenfalls gewünschte n-Selektivität zum Nonanal mit 36,8 % bis maximal 57,6 % verbesserungswürdig. Dies gilt umso mehr, als dass die Verwendung der katalytisch aktiven Zusammensetzung in technischen Prozessen eine Standzeit verlangt, welche sich in Tagen anstelle von Stunden bemisst.

Literaturbekannt ist die Synthese symmetrisch aufgebauter Bisphosphite, wie sie seit US 4 769 498 offenbart wurden und deren Verwendung in katalytisch aktiven, übergangsmetallhaltigen Zusammensetzungen zur Hydroformylierung ungesättigter Verbindungen.

Bei den in US 5 288 918 in Spalte 8 unter der allgemeinen Formel (V) offenbarten Bisphosphiten handelt es sich um symmetrische Bisphosphite. Das Bisphosphit ist selbst dann symmetrisch, wenn X¹ und X² für unterschiedliche Reste stehen, wie es in der Tabelle in Spalte 11 bei Ref.No. 2 und 3 der Fall ist.

In US 4 769 498, wie auch in US 5 723 641 werden bevorzugt symmetrisch aufgebaute Bisphosphite hergestellt und als Liganden zur Hydroformylierung verwendet. Die in der Hydroformylierung verwendeten symmetrisch aufgebauten Bisphosphitliganden werden bei tiefen Temperaturen hergestellt. Die Einhaltung dieser tiefen Temperaturen ist zwingend erforderlich, da höhere Temperaturen gemäß dieser US-Schriften zu Umlagerungen und letztlich zu unsymmetrisch aufgebauten Bisphosphiten führen würde, was aber hier nicht erwünscht ist.

Diese unsymmetrisch aufgebauten Bisphosphite weisen bei der Verwendung als Ligand in der übergangsmetallkatalysierten Hydroformylierung deutlich geringere Reaktivitäten und geringere n-Regioselektivität auf; siehe in Rhodium-catalyzed Hydroformylation, ed. by P.W.N.M. van Leeuwen et C. Claver, Kluwer Academic Publishers 2006, AA Dordrecht, NL, Seite 45-46.

Wie von van Leeuwen ausgeführt, weisen die symmetrischen Bisphosphite neben höheren Selektivitäten auch eine größere Reaktivität auf. Neben dem Bestreben einer hohen Reaktivität und n-Selektivität in Bezug auf die zu carbonylierenden, ungesättigten Verbindungen ist die Stabilität - konkret die Standzeit - der katalytisch aktiven Zusammensetzung aus jeweils verwendetem Metall, Liganden sowie weiteren Komponenten mit aktivierender Wirkung mit Blick auf die als Liganden eingesetzten Bisphophite eine ständige Aufgabe der Forschung. Dies gilt insbesondere hinsichtlich olefinhaltiger Gemische, speziell in der Hydroformylierung von Gemischen linearer Olefine.

In US 5364950, wie auch in US 5763677 und in *"*Catalyst Separation, Recovery and Recycling", herausgegeben v. D.J. Cole-Hamilton, R.P. Tooze, 2006, NL, Seiten 25-26, wird die Bildung von sogenannten *"Poisoning Phosphites"* als Neben- bzw. Ligandenabbaureaktion beschrieben. Diese *"Poisoning Phosphites"* bilden sich bei der Verwendung von arylphosphit-modifizierten Rhodium-Komplexen während der Hydroformylierungsreaktion. Hierbei kommt es im Zuge des Ligandenabbaus zu einem Austausch einer Arylgruppe durch eine Alkylgruppe des Hydroformylierungsproduktes.

Neben der Bildung der unerwünschten *"Poisoning Phosphites"* kann der Phosphitligand auch im Zuge einer Hydrolysereaktion durch die bei der Aldehydkondensation gebildeten Wasserspuren abgebaut werden. Eine Konsequenz aus diesen Abbaureaktionen der Liganden ist, dass die Konzentration an hydroformylierungsaktiven Rhodiumkomplexspezies im Laufe der Zeit abnimmt und mit einem Verlust an Reaktivität einher geht.

Es ist allgemein bekannt, dass bei einer kontinuierlichen Fahrweise der Hydroformylierung Ligand/en und - gegebenenfalls weitere Komponenten - während des Reaktionsverlaufs nachdosiert, d. h. nach Reaktionsbeginn zusätzlich zugegeben werden müssen (siehe DE 10 2008 002 187 A1).

Die technische Aufgabe der vorliegenden Erfindung ist die Bereitstellung neuer Liganden, welche in der Hydroformylierung von ungesättigten Verbindungen nicht die aus dem Stand der Technik zuvor aufgezeigten Nachteile, sondern die folgenden Eigenschaften aufweisen:
1.) eine hohe Aktivität, und
2.) eine hohe n-Regioselektivität in Bezug auf die Hydroformylierung und
3.) eine hohe Standzeit.

Eine hohe Standzeit bedeutet, dass die hydroformylierungsaktive Zusammensetzung, welche die Liganden neben weiteren Komponenten umfasst, eine geringe Tendenz zum Abbau dieser Liganden und/oder dem Zerfall dieser Liganden in hydroformylierungs-inhibierende Komponenten, wie z.B. die sogenannten *"Poisoning Phosphites"* aufweist.

Die Aufgabe wird gelöst durch ein Gemisch umfassend die Verbindungen (Ia) und (IIa):
Gemisch umfassend die Verbindungen (Ia) und (IIa): 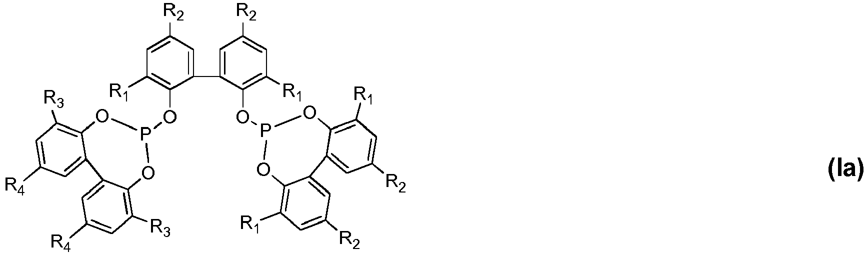 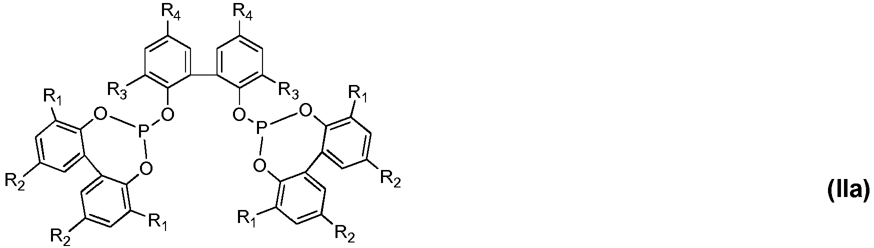 wobei
   R1 ausgewählt ist aus -Me, -tBu, -OMe;
   R2 ausgewählt ist aus -Me, -tBu, -OMe;
   R3 ausgewählt ist aus -Me, -tBu, -OMe;
   R4 ausgewählt ist aus -Me, -tBu, -OMe;
   unter der Voraussetzung dass,
   für den Fall dass R1 gleich R3 ist, R2 nicht gleich R4 ist,
   für den Fall dass R2 gleich R4 ist, R1 nicht gleich R3 ist,
   und P weitere Bindungen eingehen kann.

Durch die Voraussetzungen: für den Fall dass R1 gleich R3 ist, R2 nicht gleich R4 ist, für den Fall dass R2 gleich R4 ist, R1 nicht gleich R3 ist, wird ausgeschlossen, dass alle drei Biphenole gleich substituiert sind.
Im Falle von Ia liegt ein unsymmetrisches Bisphosphit vor, wohingegen IIa ein symmetrisches Bisphosphit darstellt. Das Gemisch weist somit ein symmetrisches und ein unsymmetrisches Bisphosphit auf.

Normalerweise werden im Stand der Technik möglichst reine Liganden in der Hydroformylierungsreaktion eingesetzt, da das jeweils andere Isomer starke negative Einflüsse auf die Gesamtperformance des Systems ausübt. In der Regel würde das unsymmetrische Isomer als Nebenkomponente vorliegen, da ausschließlich symmetrische Liganden in der Hydroformylierung eingesetzt werden,

In Rhodium-catalyzed Hydroformylation, ed. by P.W.N.M. van Leeuwen et C. Claver, Kluwer Academic Publishers 2006, AA Dordrecht, NL, Seite 45-46, Tabelle 2 sind die Hydroformylierungsergebnisse des symmetrischen Biphephosliganden und seines unsymmetrischen Isomers beschrieben. Dabei geht deutlich hervor, dass sich der symmetrische Biphephosligand (in der Literaturstelle Ligand 5a) durch eine deutlich höhere n/i-Selektivität und eine höhere Aktivität auszeichnet als sein unsymmetrisches Isomer (in der Literaturstelle Ligand 7). In der Hydroformylierungsreaktion von Propen weist der symmetrische Ligand eine n/i-Selektivität von 53 und eine Reaktionsrate von 402 auf, wohingegen der unsymmetrische Ligand lediglich eine n/i-Selektivität von 1.2 und eine Reaktionsrate von 280 auf. Würde man nun ein Gemisch beider Liganden einsetzen, so würde dies zu deutlich schlechteren Ausbeuten und n/i-Selektivitäten führen. Eine deutlich schlechtere Gesamtperformance konnte auch mit der Isomeren-Mischung aus den Liganden (7) und (8) verzeichnet werden. Verwendet man nun die
erfindungsgemäße Isomerenmischung in der Hydroformylierung, so ist dies nicht der Fall, und das andere Isomer kann als Nebenkomponente im Isomerengemisch vorliegen ohne die Gesamtperformance des Systems negativ zu beeinflussen. Dies ist besonders vorteilhaft, da somit während der Ligandenherstellung keine weiteren Aufreinigungsschritte notwendig sind, um ein Isomer mit einer 100%igen Reinheit zu erhalten. Dies ist besonders günstig, da jeder weitere Aufreinigungsschritt in der Ligandenherstellung zu einer Verteuerung desselbigen führt. In der Regel werden für diese Aufreinigungen verschiedenen Lösungsmittel verwendet und es sind unter Umständen verschiedenen Aufreinigungen notwendig, wie beispielsweise Umkristallisationen, die zwangsläufig zu Produktverlusten führen. Dies führt wiederum dazu, dass der Ligand in seiner Herstellung deutlich teurer wird und das wiederum wirkt sich negativ auf die Gesamtwirtschaftlichkeit eines großtechnischen Prozesses aus. Somit ist es besonders vorteilhaft, wenn es möglich ist, auf teure Aufreinigungsschritte zu verzichten und entsprechende Isomerenmischungen in einem großtechnischen Hydroformylierungsprozess einzusetzen.

In einer Ausführungsform liegt der Gehalt an Verbindung (Ia) in einem Bereich von 0,5 bis 99,5 Massen-%, der Gehalt an Verbindung (IIa) in einem Bereich von 0,5 bis 99,5 Massen-%.
Die beiden Verbindungen (Ia) und (IIa) addieren sich zu 100 Massen-%.

In einer Ausführungsform umfasst das Gemisch die Verbindungen (Ib) und (IIb): 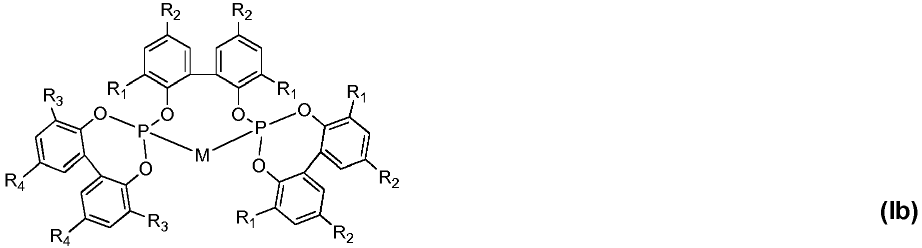 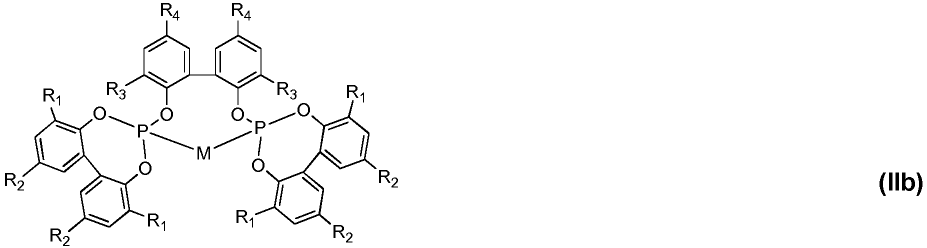 wobei M ausgewählt ist aus Fe, Ru, Os, Co, Rh, Ir, Ni, Pd, Pt,
und M zusätzliche Bindungen eingehen kann.

In einer Ausführungsform liegt der Gehalt an Verbindung (Ib) in einem Bereich von 0,5 bis 99,5 Massen-%, der Gehalt an Verbindung (IIb) in einem Bereich von 0,5 bis 99,5 Massen-%.
Die beiden Verbindungen (Ib) und (IIb) addieren sich zu 100 Massen-%.

In einer Ausführungsform umfasst das Gemisch die Verbindungen (Ic) und (IIc): 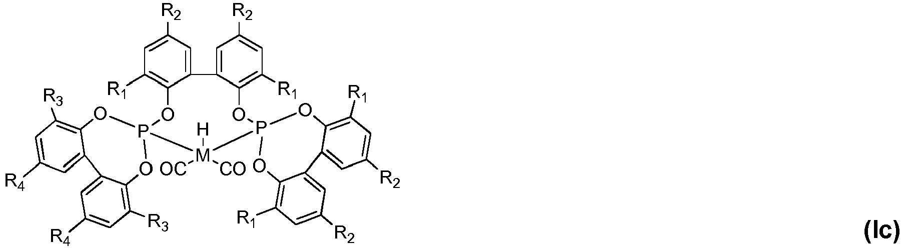 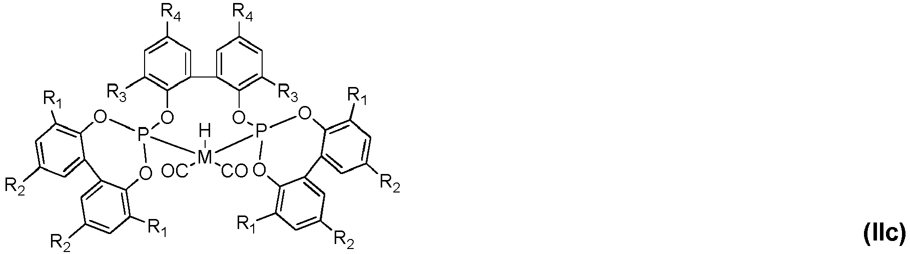 wobei M ausgewählt ist aus Fe, Ru, Os, Co, Rh, Ir, Ni, Pd, Pt.

In einer Ausführungsform liegt der Gehalt an Verbindung Ic in einem Bereich von 0,5 bis 99,5 Massen-%, der Gehalt an Verbindung IIc in einem Bereich von 0,5 bis 99,5 Massen-%.
Die beiden Verbindungen Ic und IIc addieren sich zu 100 Massen-%.

In einer Ausführungsform umfasst das Gemisch zusätzlich mindestens eine Verbindung (Ia) oder (IIa), die nicht an M gebunden ist.

In einer Ausführungsform steht M für Rh.

In einer Ausführungsform ist R1 -Me, und R3 nicht -Me.

In einer Ausführungsform ist R2 -Me, und R4 nicht -Me.

In einer Ausführungsform sind R1 und R2 -Me.

In einer Ausführungsform ist R1 -tBu, und R3 nicht -tBu.

In einer Ausführungsform ist R2 -OMe, und R4 nicht -OMe.

Die verschiedenen Gemische können entweder direkt aus der Synthese hervorgehen, d.h. das beide Isomere (Ia) und (IIa) während ein und derselben Synthese generiert werden, oder aus den reinen Verbindungen der Formel (Ia) und (IIa) im Anschluss an die Synthese gemischt werden.

In einer bevorzugten Ausführungsform weisen die Verbindungen die Strukturen (1Ia) und (2IIa) auf:

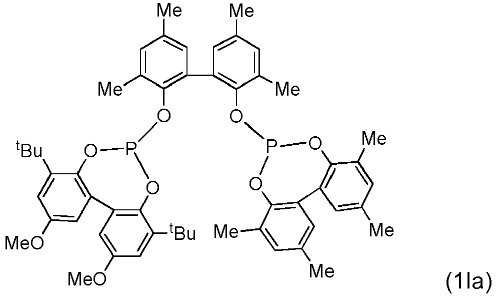

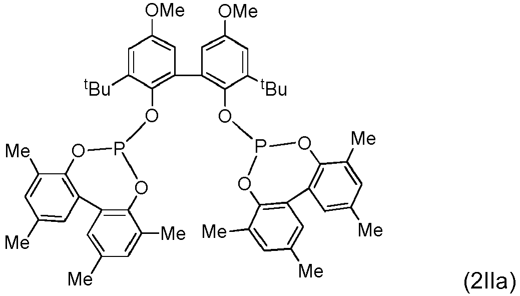

In einer weiteren bevorzugten Ausführungsform weisen die Verbindungen die Strukturen (3Ia) und (4IIa) auf:

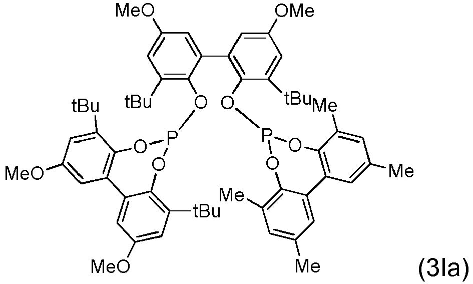

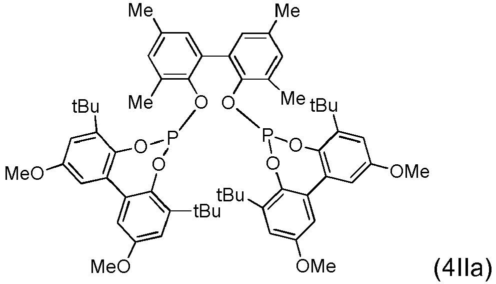

Exemplarisch für die verschiedenen Reste R sind die Verbindungen (1Ia), (2IIa). (3Ia) und (4IIa) in der nachfolgenden Tabelle 1 zusammengefasst.

**Tabelle 1:**

| | Isomer | R1 | R2 | R3 | R4 |
|---|---|---|---|---|---|
| (1Ia) | (Ia) | -Me | -Me | -tBu | -OMe |
| (2IIa) | (IIa) | -Me | -Me | -tBu | -OMe |
| (3Ia) | (Ia) | -tBu | -OMe | -Me | -Me |
| (4IIa) | (IIa) | -tBu | -OMe | -Me | -Me |

Die Erfindung umfasst folgende Gegenstände:
a) Gemische Bisphosphite der Formeln (Ia) und (IIa);
b) Verfahren zu deren Herstellung;
c) Metall-Gemische der Formeln (Ib) und (IIb), wobei M ein Metall der 4. bis 10. Gruppe des Periodensystems der Elemente (Fe, Ru, Os, Co, Rh, Ir, Ni, Pd, Pt) darstellt und zusätzliche Bindungen eingehen kann und die Konstitutionsisomeren der Formeln (Ia) und (IIa) vorliegen, die nicht an das Metall M gebunden sind; 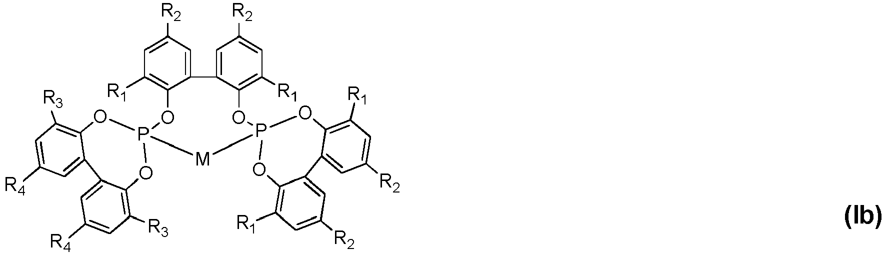 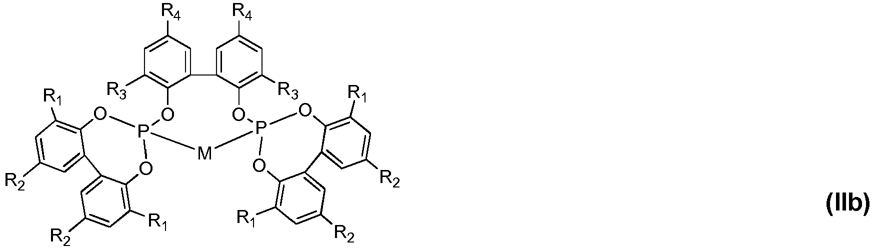 wobei M ausgewählt ist aus Fe, Ru, Os, Co, Rh, Ir, Ni, Pd, Pt,
   und M zusätzliche Bindungen eingehen kann.
d) Zusammensetzungen, enthaltend die unter a) genannten Konstitutionsisomeren, Metalle der 4. bis 10. Gruppe des Periodensystems der Elemente (Fe, Ru, Os, Co, Rh, Ir, Ni, Pd, Pt) sowie freie, d.h. ungebundene Bisphosphite der Formeln (1a) und (2a) und zumindest eine weitere Komponente, ausgewählt aus der Gruppe, welche Basen, organische Amine, Epoxide, Ionenaustauscher, Puffersysteme umfasst; 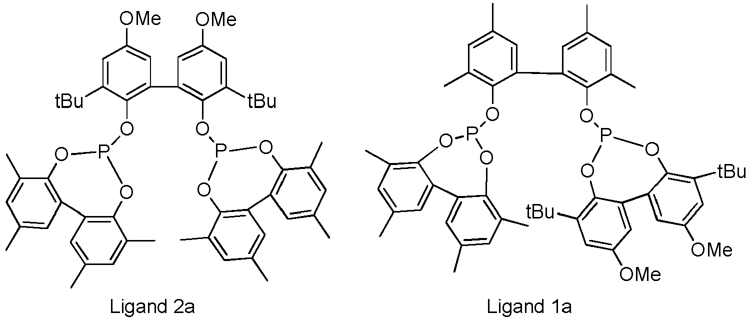
e) Verfahren zur Hydroformylierung ungesättigter Verbindungen und deren Gemischen unter Verwendung von Zusammensetzungen nach d), eines Gasgemisches bestehend aus Kohlenmonoxid und Wasserstoff, ungesättigten Verbindungen und deren Gemischen unter den für eine Hydroformylierung erforderlichen Reaktionsbedingungen.

Neben dem Gemisch wird auch eine Zusammensetzung beansprucht, welche ein solches umfasst.

Zusammensetzung umfassend:
- ein zuvor beschriebenes Gemisch,
- eine weitere Komponente ausgewählt aus: Basen, organische Amine, Epoxide, Pufferlösungen, Ionenaustauscher.

In einer bevorzugten Ausführungsform werden als weitere Komponenten sterisch gehinderte sekundäre Amine eingesetzt.

Es können auch Gemische, enthaltend zwei oder mehrere sterisch gehinderte Amine, eingesetzt werden.

Die Zusammensetzung umfasst ein zuvor beschriebenes Gemisch, welche zusätzlich zu dem Gemisch zumindest ein Amin mit einer 2,2,6,6-Tetramethylpiperidineinheit aufweist.

Insbesondere wird im erfindungsgemäßen Verfahren das Amin mit der Formel (11), Sebacinsäuredi-4-(2,2,6,6-tetramethylpiperidinyl)ester, bevorzugt eingesetzt.

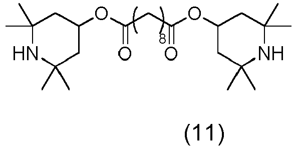

Ein besonders bevorzugtes Metall in der erfindungsgemäßen Zusammensetzung ist Rhodium.

Neben dem Gemisch wird auch ein Verfahren zu dessen Herstellung beansprucht.

Verfahren zur Herstellung eines zuvor beschriebenen Gemisches,
umfassend die Verfahrensschritte:
a) oxidative Kopplung gemäß Reaktionsschema A: 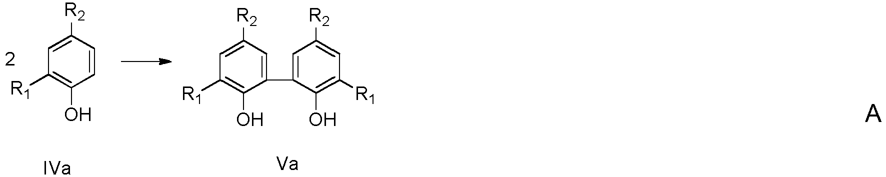
b) oxidative Kopplung gemäß Reaktionsschema B: 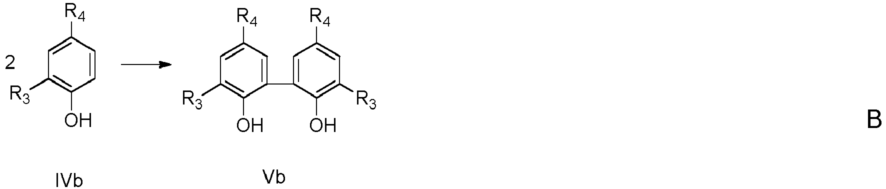
c) Umsetzung des Produktes aus a) mit PCl₃ gemäß Reaktionsschema C: 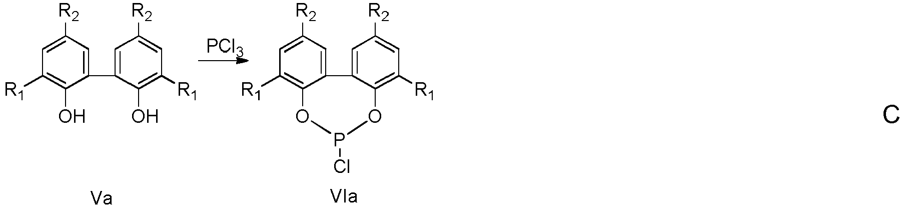
d) Umsetzung des Produktes aus b) mit dem Produkt aus c) zu einem Gemisch nach Anspruch 1.

Ein besonderer Vorteil der vorliegenden Erfindung liegt in der Verwendung der zuvor beschriebenen Gemische (Ia) und (IIa), insbesondere (1Ia) und (2IIa), in der Hydroformylierung darin begründet, dass die erfindungsgemäße Verwendung der Mischung der konstitutionsisomeren Bisphophite anstelle eines reinen Verbindungen eine aufwendige und kostspielige Auftrennung der konstitutionsisomeren Bisphosphite überflüssig macht.

Aus dem Stand der Technik erwartete man eine herabgesetzte Reaktivität sowie geringere n/i-Selektivität aufgrund der Anwesenheit des unsymmetrischen Bisphosphits (Ia), insbesondere dessen Derivats (Ic). Wie in den nachfolgenden Hydroformylierungsexperimenten offenbart wird, weisen die Verbindungen Bisphosphite (1Ia) und (2IIa) überraschenderweise neben hohen Reaktivitäten und n/i-Selektivitäten eine deutlich erhöhte Standzeit gegenüber den aus dem Stand der Technik bekannten Bisphosphiten auf.

In einer Ausführungsform umfasst das Verfahren zusätzlich den Verfahrensschritt:
e) Umsetzung mit M zu (Ib) und (IIb), wobei M ausgewählt ist aus Fe, Ru, Os, Co, Rh, Ir, Ni, Pd, Pt.

Des Weiteren wird auch die Verwendung des Gemisches als Katalysator in einer Hydroformylierungsreaktion von ungesättigten Verbindungen und deren Gemischen beansprucht. Die Formulierung "als Katalysator" ist hierbei so zu verstehen, dass die Verbindungen als Liganden für einen Metallkomplex eingesetzt werden, welcher dann die entsprechende Reaktion katalysiert.

Diese definierten Gemische der Bisphosphite, bestehend aus den Verbindungen der Formel (Ia) und (IIa), können beispielsweise direkt zu Beginn einer Hydroformylierungsreaktion vorgelegt werden. Diese Vorgehensweise unterscheidet sich somit von dem allgemeinen Vorgehen bei einer Stabilitätsuntersuchung, bei der ein definiertes Isomer vorgelegt wird und die weiteren Verbindungen sich erst im Reaktionsverlauf bilden.

Des Weiteren wird ein Verfahren zur Hydroformylierung von ungesättigten Verbindungen und deren Gemischen beansprucht.

Verfahren zur Hydroformylierung von ungesättigter Verbindungen und deren Gemischen unter Verwendung:
i) einer zuvor beschriebenen Zusammensetzung,
ii) eines Gasgemisches umfassend Kohlenmonoxid und Wasserstoff.

Die ungesättigten Verbindungen, welche in dem erfindungsgemäßen Verfahren hydroformyliert werden, umfassen Kohlenwasserstoffgemische, die in petrochemischen Verarbeitungsanlagen anfallen. Hierzu gehören beispielsweise sogenannte C₄-Schnittte. Typische Zusammensetzungen von C₄-Schnitten, aus denen der größte Teil der mehrfach ungesättigten Kohlenwasserstoffe entfernt worden ist und die im erfindungsgemäßen Verfahren eingesetzt werden können, sind in der folgenden Tabelle 1 aufgelistet (siehe DE 10 2008 002188).

**Tabelle 2:**

| | Dampfspaltanlage | | Dampfspaltanlage | | Katalytische Spaltanlage | |
|---|---|---|---|---|---|---|
| Komponente | HCC₄ | HCC₄ / SHP | Raff. I | Raff. I / SHP | CC₄ | CC₄ / SHP |
| Isobutan [Massen-%] | 1 - 4.5 | 1 - 4.5 | 1.5 - 8 | 1.5 - 8 | 37 | 37 |
| n-Butan [Massen-%] | 5 - 8 | 5 - 8 | 6 - 15 | 6 - 15 | 13 | 13 |
| E-2-Buten [Massen-%] | 18 - 21 | 18 - 21 | 7 - 10 | 7 - 10 | 12 | 12 |
| 1-Buten [Massen-%] | 35 - 45 | 35 - 45 | 15 - 35 | 15 - 35 | 12 | 12 |
| Isobuten [Massen-%] | 22 - 28 | 22 - 28 | 33 - 50 | 33 - 50 | 15 | 15 |
| Z-2-Buten [Massen-%] | 5 - 9 | 5 - 9 | 4 - 8 | 4 - 8 | 11 | 11 |
| 1,3-Butadien [Massen-ppm] | 500 - 8000 | 0 - 50 | 50 - 8000 | 0 - 50 | < 10000 | 0-50 |

Erläuterung:
- HCC₄: typisch für eine C₄ Mischung, die aus dem C₄-Schnitt einer Dampfspaltanlage (High Severity) nach der Hydrierung des 1,3-Butadiens ohne zusätzliche Moderation des Katalysators erhalten wird.
- HCC₄ / SHP: Zusammensetzung HCC₄, bei dem Reste an 1,3-Butadien in einem Selektivhydrierungsprozess/SHP weiter reduziert wurden.
- Raff. I (Raffinat I): typisch für eine C₄ Mischung, die aus dem C₄-Schnitt einer Dampfspaltanlage (High Severity) nach der Abtrennung des 1,3-Butadiens, beispielsweise durch eine NMP-Extraktivrektifikation, erhalten wird.
- Raff. I / SHP: Zusammensetzung Raff. I, bei dem Reste an 1,3-Butadien in einem Selektivhydrierungsprozess/SHP weiter reduziert wurden.
- CC₄: typische Zusammensetzung eines C₄-Schnitts, das aus einer katalytischen Spaltanlage erhalten wird.
- CC₄ / SHP: Zusammensetzung eines C₄-Schnitts, bei dem Reste an 1,3-Butadien in einem Selektivhydrierungsprozess/SHP weiter reduziert wurden.

In einer Variante des Verfahrens ist die ungesättigte Verbindung oder deren Gemisch ausgewählt aus:
- Kohlenwasserstoffgemischen aus Dampfspaltanlagen;
- Kohlenwasserstoffgemischen aus katalytisch betriebenen Spaltanlagen, wie z.B. FCC-Spaltanlagen;
- Kohlenwasserstoffgemischen aus Oligomerisierungsprozessen in homogener Phase sowie heterogenen Phasen, wie z.B. dem OCTOL-, DIMERSOL.-, Fischer-Tropsch-, Polygas-, CatPoly-, InAlk-, Polynaphtha-, Selectopol-, MOGD-, COD-, EMOGAS-, NExOCTANE- oder SHOP-Prozess;
- Kohlenwasserstoffgemischen umfassend mehrfach ungesättigte Verbindungen;
- ungesättigten Carbonsäurederivaten.

In einer Variante des Verfahrens weist das Gemisch ungesättigte Verbindungen mit 2 bis 30 Kohlenstoffatomen auf.

In einer besonderen Variante des Verfahrens weist das Gemisch ungesättigte Verbindungen mit 2 bis 8 Kohlenstoffatomen auf.

In einer weiteren Variante des Verfahrens weist das Gemisch mehrfach ungesättigte Kohlenwasserstoffe auf. In einer besonderen Ausführungsform umfasst das Gemisch Butadiene.

Die ungesättigten Verbindungen, welche in dem erfindungsgemäßen Verfahren hydroformyliert werden, umfassen weiterhin ungesättigte Carbonsäurederivate. In einer besonderen Ausführungsform sind diese ungesättigten Carbonsäurederivate ausgewählt unter Fettsäureestern.

Die Durchführung des erfindungsgemäßen Verfahrens erfolgt in unterschiedlichen Ausführungsformen, welche in den Beispielen im Detail offenbart werden.

Das erfindungsgemäße mehrphasige Reaktionsgemisch umfasst neben einem aus Kohlenmonoxid und Wasserstoff bestehenden Gasgemisch mindestens eine ungesättigte Verbindung, wie sie zuvor offenbart wurde und umfasst neben Kohlenwasserstoffgemischen, welche aus Dampfspalt-, katalytisch betriebenen Spaltanlagen oder Oligomerisierungsprozessen stammen oder andere Quellen von einfach ungesättigten und/oder mehrfach ungesättigten Kohlenstoffverbindungen oder ungesättigte Carbonsäurederivate beinhalten, mindestens ein Hydroformylierungsprodukt dieser ungesättigten Verbindungen, wie sie in den nachfolgenden Beispielen aufgeführt sind und die jeweils verwendete Zusammensetzung, wie sie zuvor offenbart wurde.

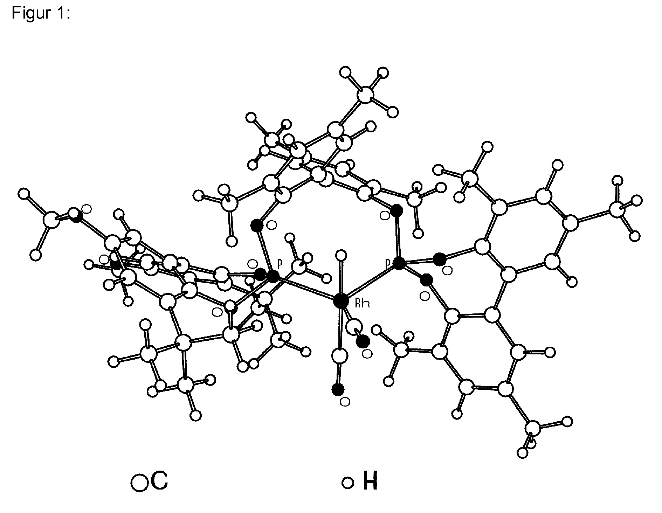

Die Figur 1 zeigt die berechnete Komplexverbindung (Ic) mit R1=Me, R2=Me, R3=tBu, R4=OMe und M=Rh.

Die erfindungsgemäßen Komplexverbindung der Formeln (Ic) und (IIc) werden in-situ während der Hydroformylierungsreaktion gebildet.
In einer besonderen Ausführungsform der Erfindung liegen die Komplexverbindungen (Ic) und (IIc) neben dem ungebundenen Bisphosphit vor.
Die Charakterisierung des Hydridocarbonylkomplexes Ic mit Rhodium als Metall erfolgte mittels theoretischer Berechnungen. Das Ergebnis ist in der Figur 1 dargestellt.

Die Strukturberechnung wurde mit dem BP86-Funktional und dem def-SV(P)-Basissatz durchgeführt.
Die Strukturberechnungen für die Modellstrukturen erfolgten mit dem Turbomole-Programmpaket (R. Ahlrichs, M. Bär, M. Häser, H. Horn, C. Kölmel, Chem. Phys. Lett., 1989, 162, 16; TURBOMOLE V6.3 2011, a development of University of Karlsruhe and Forschungszentrum Karlsruhe GmbH, 1989-2007, TURBOMOLE GmbH, since 2007. http://www.turbomole.com) auf Basis der Dichtefunktionaltheorie (DFT). Verwendet wurde das BP86-Funktional (S. H. Vosko, L. Wilk, M. Nusair, Can. J. Phys. , 1980, 58, 1200; A. D. Becke, Phys. Rev. A, 1988, 38, 3098; J. Perdew, Phys. Rev. B, 1986, 33, 8822) und der def-SV(P)-Basissatz (A. Schäfer, H. Horn and R. Ahlrichs, J. Chem. Phys., 1992, 97, 2571).

### Beispiele

### Synthese des Ligandengemisches (11a) und (211a)

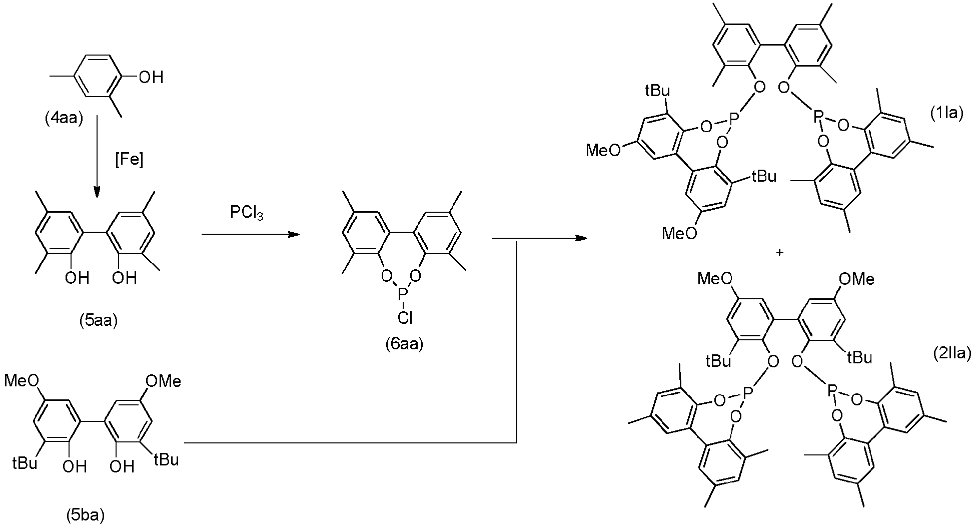

**Abkürzungen:**
- VE-Wasser =: demineralisiertes Wasser
- KPG =: Kerngezogenes Präzisions-Glasgerät
- ACN =: Acetonitril
- EtOAc =: Ethylacetat
- DMAB =: Dimethylaminobutan
- NMP =: N-Methylpyrrolidon
- ÖV =: Ölvakuum
- acac =: acetylacetonat
- NEt₃ =: Triethylamin
- TIPB =: 1,2,4,5-Tetraisopropylbenzol

### Synthese des 2,2'-Bis(3,5-dimethylphenol) (5aa)

Das als Vorstufe eingesetzte Biphenol (5aa) wurde nach folgender Synthesevorschrift hergestellt.

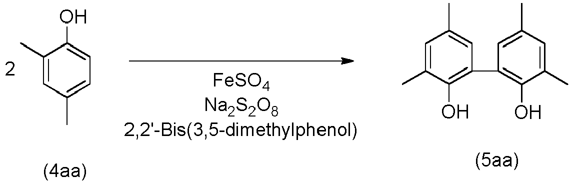

In einem 500 ml Schlenk mit KPG-Rührer, Zwischenaufsatz und Glasrührer wurden 1,42 g (0,005 mol) Eisen(II)-sulfatheptahydrat und 12,35 g (0,1 mol) 2,4-Dimethylphenol in 150 ml VE-Wasser und 5 ml Cyclohexan vorgelegt und auf 40 °C erwärmt.
In einem 100 ml Becherglas löste man 25.36 g (0,146 mol) Natriumperoxodisulfat in 80 ml VE-Wasser. Zum Start der Reaktion wurde eine kleine Portion Na₂S₂O₈-Lösung zum Phenol gegeben. Anschließend wurde alle 10 min eine kleinere Portion der Lösung hinzugegeben. Nach 30 min war die Na₂S₂O₈-Lösung hinzugegeben.
Nach einer Reaktionszeit von 5h wurde zur Reaktionslösung 300 ml Cyclohexan und 200 ml Wasser hinzugegeben, 20 min rühren gelassen, dann warm in den Scheidetrichter überführt.

Die organische Phase wurde abgetrennt und bis zur Trockene eingeengt. Das Produkt konnte in 69%iger Ausbeute (10,6 g) erhalten werden.

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).
Die Charakterisierung des Produktes erfolgte mittels NMR-Spektroskopie. Chemische Verschiebungen werden in ppm angegeben. Die Referenzierung der ³¹P-NMR-Signale erfolgte gemäß: SR_{31P} = SR_{1H} * (BF_{31P} / BF_{1H}) = SR_{1H} * 0,4048. (Robin K. Harris, Edwin D. Becker, Sonia M. Cabral de Menezes, Robin Goodfellow, and Pierre Granger, Pure Appl. Chem., 2001, 73, 1795 - 1818; Robin K. Harris, Edwin D. Becker, Sonia M. Cabral de Menezes, Pierre Granger, Roy E. Hoffman and Kurt W. Zilm, Pure Appl. Chem., 2008, 80, 59-84). Mittels der ³¹P-NMR wurde das Verhältnis der beiden Liganden (Ligand 1Ia und Ligand 2IIa) zueinander erung des Isomerengemisches bestehend aus (1Ia) und (2IIa) und Amins (11) wurde eine iert, wohingegen für den symmetrischen Liganden (2IIa) nur ein Phosphorsignal zu erwarten ist.

### Synthese des 2,2'-Bis-(3,5-dimethyphenol)chlorophosphits

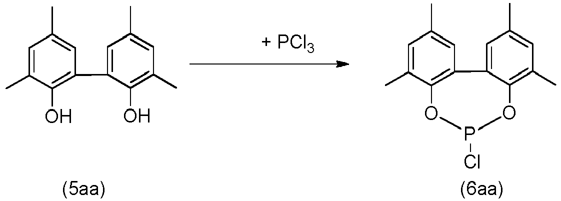

In einem sekurierten 2 L Schlenk mit Magnetrührer wurden 440 ml Phosphortrichlorid vorgelegt. In einem zweiten sekurierten 1 L Schlenk wurden 120 g 2,2-Bis-(3,5-dimethylphenol) eingewogen und unter Rühren 500 ml getrocknetes Toluol hinzugefügt. Die Biphenol-Toluol-Suspension wurde innerhalb von 4 h bei 63 °C zum Phosphortrichlorid dosiert. Nach vollständiger Zugabe wurde die Reaktionsmischung über Nacht bei Temperatur gerührt. Am nächsten Morgen wurde die Lösung in der Wärme (45 °C) eingeengt und das Produkt konnten in 96,5%iger Ausbeute (153 g) erhalten werden. ³¹P-NMR: 175,59 (94,8% 2,2'-Bis-(3,5-dimethylphenol)chlorophosphit), 4,4% diverse PCI-Verbindungen, 0,8% P-H-Verbindung.

### Erfindungsgemäße Synthesevariationen zur Herstellung des Gemisches aus Liganden (1Ia) und (2IIa):

### Variante 1: ACN/NEt₃

In einem 1000 ml Schlenk wurde unter Schutzgas 38,75 g (0,121 mol) 2,2'-Bis-(3,5-dimethylphenyl)chlorophosphit in 150 ml entgastem ACN gelöst und auf 35°C erwärmt. In einem zweiten Schlenk (500 ml) wurden 20,1 g (0,056 mol) 3,3'-Di-tert.-butyl-5,5'-dimethoxy-[1,1'-biphenyl]-2,2'diol in 150 ml entgastem ACN gelöst und unter Rühren mit 40,9 ml entgastem Triethylamin (0,29 mol) versetzt. Dann wurde langsam die Biphenol/Triethylamin-Lösung zu der Chlorophosphitlösung getropft. Nach einer Nachreaktionszeit von 1h wurde die Reaktionslösung über Nacht bei 45 °C gerührt.
Anschließend wurde die Lösung filtriert und der Feststoff dreimal mit 100 ml warmem (45 °C) ACN gewaschen. Das Zielprodukt konnte als weißer Feststoff (43,3 g, 86%) erhalten werden. ³¹P-NMR (202,4 MHz, toluene-d₈): 142,5 und 140,9 (95,4%) 139,2 (4,6%).

### Variante 2: EtOAc/NEt₃

In einem 100 ml Schlenk wurde unter Schutzgas 7,3 g (21,0 mmol) 2,2'-Bis-(3,5-dimethylphenyl)chlorophosphit in 15 ml entgastem Ethylacetat gelöst und auf 35°C erwärmt. In einem zweiten Schlenk (100 ml) wurden 3,9 g (9,5 mmol) 3,3'-Di-tert.-butyl-5,5'-dimethoxy-[1,1'-biphenyl]-2,2'diol in 7,0 ml NEt₃ gelöst. Anschließend wurde die Biphenol/Triethylamin-Lösung langsam innerhalb von 20 Minuten zu der Chlorophosphitlösung getropft. Die Lösung wurde eine weitere Stunde bei 35 °C und anschließend über Nacht bei 45 °C gerührt.
Am nächsten Tag wurde die Lösung filtriert und der Feststoff dreimal mit ACN gewaschen. Das Zielprodukt konnte als weißer Feststoff (6,7 g, 78%) erhalten werden. ³¹P-NMR (202,4 MHz, toluene-d₈): 142,5 und 140,9 (91,3%), 139,5 (8,7%).

### Variante 4: ACN/DMAB (Dimethylaminobutan)

In einem 100 ml Schlenk wurde unter Schutzgas 6 g (19,0 mmol) 2,2'-Bis-(3,5-dimethylphenyl)chlorophosphit in 20 ml entgastem ACN gelöst und auf 35°C erwärmt. In einem zweiten Schlenk (50 ml) wurden 3,4 g (9,0 mmol) 3,3'-Di-tert.-butyl-5,5'-dimethoxy-[1,1'-biphenyl]-2,2'diol in 15 ml Dimethylaminobutan (DMAB) gelöst und anschließend langsam zu der Chlorophosphitlösung getropft. Die Reaktion wurde bei 35 °C über Nacht rühren gelassen. Am nächsten Tag wurde die Lösung filtriert und der Feststoff zweimal mit ACN gewaschen. Das Zielprodukt konnte als weißer Feststoff (5,3 g, 66%) erhalten werden. ³¹P-NMR (202,4 MHz, toluene-d₈): 142,8 und 141,2 (97,5%), 139,4 (2,5%).

### Variante 5: ACN/NMP (N-Methylpyrrolidon)

In einem 100 ml Schlenk wurde unter Schutzgas 6 g (19,0 mmol) 2,2'-Bis-(3,5-dimethylphenyl)chlorophosphit in 20 ml entgastem ACN gelöst und auf 35°C erwärmt. In einem zweiten Schlenk (50 ml) wurden 3,4 g (9,0 mmol) 3,3'-Di-tert.-butyl-5,5'-dimethoxy-[1,1'-biphenyl]-2,2'diol in 9,4 ml N-Methylpyrrolidon (NMP) gelöst und langsam zu der Chlorophosphitlösung getropft. Die Reaktion wurde bei 35°C über Nacht rühren gelassen. Anschließend wurde die Lösung filtriert und der Feststoff zweimal mit ACN gewaschen. Das Zielprodukt konnte als weißer Feststoff (3,4 g, 42%) erhalten werden. ³¹P-NMR (202,4 MHz, toluene-d₈): 142,2 und 141,0 (96,1%), 139,8 (3,9%).

### Variante 6: ACN/Diisopropylethylamin

In einem 500 ml Schlenk wurde unter Schutzgas 19,4 g (61,0 mmol) 2,2'-Bis-(3,5-dimethylphenyl)chlorophosphit in 75 ml entgastem ACN suspendiert. In einem zweiten Schlenk (250 ml) wurden 10,5 g (28,5 mmol) 3,3'-Di-tert.-butyl-5,5'-dimethoxy-[1,1'-biphenyl]-2,2'diol in 75 ml Acetonitril und 39 ml Diisopropylethylamin suspendiert und langsam zu der Chlorophosphitlösung gegeben. Die Reaktion wurde über Nacht rühren gelassen. Anschließend wurde die Lösung filtriert und der Feststoff dreimal mit ACN gewaschen. Das Zielprodukt konnte als weißer Feststoff (14,6 g, 57%) erhalten werden. ³¹P-NMR (202,4 MHz, toluene-d₈): 142,2 und 141,1 (76,8%), 139,1 (23,2%).

### Variante 7: Toluol/ Dimethylaminobutan

In einem 100 ml Schlenk wurde unter Schutzgas 7,7 g (24,0 mmol) 2,2'-Bis-(3,5-dimethylphenyl)chlorophosphit in 15 ml entgastem Toluol gelöst und auf 35°C erwärmt. In einem zweiten Schlenk (50 ml) wurden 3,4 g (9,0 mol) 3,3'-Di-tert.-butyl-5,5'-dimethoxy-[1,1'-biphenyl]-2,2'diol in 15 ml Dimethylaminobutan (DMAB) gelöst und langsam zu der Chlorophosphitlösung getropft. Die Reaktion wurde bei 45 °C 4 Tage rühren gelassen. Im Anschluss daran wurde die Lösung nach weiterer Zugabe von 120 ml Toluol für 30 Minuten auf 75°C erwärmt.
Anschließend wurde die Lösung filtriert, das Filtrat bis zur Trockene eingeengt und getrocknet. Das Zielprodukt konnte als weißer Feststoff (7,2 g, 88%) erhalten werden. ³¹P-NMR (202,4 MHz, toluene-d₈): 142,5 und 140,9 (91,4%), 139,2 (8,6%).

### Variante 8: Variation der Aminmenge (ACN/NEt₃)

A: In einem 500 ml Schlenk wurde unter Schutzgas 17,81 g (0,073 mol) 2,2'-Bis-(3,5-dimethylphenyl)chlorophosphit in 60 ml entgastem ACN versetzt und auf 35°C erwärmt. In einem zweiten Schlenk (250 ml) wurden 9,91 g (0,0276 mol) 3,3'-Di-tert.-butyl-5,5'-dimethoxy-[1,1'-biphenyl]-2,2'diol in 60 ml entgastem ACN gelöst und unter Rühren mit 38,4 ml entgastem Triethylamin versetzt. Diese Biphenol/Triethylamin-Lösung wurde dann langsam zu der Chlorophosphitlösung getropft. Nach einer Nachreaktionszeit von 1h wurde die Reaktionslösung über Nacht bei 35 °C gerührt.
Anschließend wurde die Lösung filtriert und der Feststoff mit ACN gewaschen. Das Zielprodukt konnte als weißer Feststoff (27,8 g, 86%) erhalten werden. ³¹P-NMR (202,4 MHz, toluene-d₈): 142,8 und 141,2 (91,6%), 139,4 (8,4%).

**B:** In einem 250 ml Schlenk wurde unter Schutzgas 1,57 g (5,1 mmol) 2,2'-Bis-(3,5-dimethylphenyl)chlorophosphit in 7 ml entgastem ACN versetzt und auf 35 °C erwärmt. In einem zweiten Schlenk (100 ml) wurden 0,932 g (2,6 mmol) 3,3'-Di-tert.-butyl-5,5'-dimethoxy-[1,1'-biphenyl]-2,2'diol in 9 ml entgastem ACN gelöst und unter Rühren mit 2,09 ml entgastem Triethylamin versetzt. Dann wurde langsam die Biphenol/Triethylamin-Lösung zu der Chlorophosphitlösung getropft. Nach einer Nachreaktionszeit von 1h wurde die Reaktionslösung über Nacht bei 35 °C gerührt.
Anschließend wurde die Lösung filtriert und der Feststoff mit ACN gewaschen. Das Zielprodukt konnte als weißer Feststoff in 40%iger Ausbeute erhalten werden. ³¹P-NMR (202,4 MHz, toluene-d₈): 142,8 und 141,8 (92,4 %), 139,3 (7,6%).

### Variante 9: Verkürzte Reaktionszeiten

### A (8 Stunden): EtOAc/NEt₃

In einem 100 ml Schlenk wurde unter Schutzgas 8 g (25,0 mmol) 2,2'-Bis-(3,5-dimethylphenyl)chlorophosphit in 20 ml entgastem Ethylacetat gelöst und auf 45°C erwärmt. In einem zweiten Schlenk (50 ml) wurden 4,48 g (12,5 mmol) 3,3'-Di-tert.-butyl-5,5'-dimethoxy-[1,1'-biphenyl]-2,2'diol in 20 ml Ethylacetat und 8,0 ml NEt₃ suspendiert. Anschließend wurde die Biphenol/Triethylamin-Suspension langsam innerhalb von 30 Minuten zu der Chlorophosphitlösung getropft. Die Lösung wurde acht Stunden bei 45 °C gerührt. Anschließend wurde die Lösung filtriert. Das Zielprodukt konnte als weißer Feststoff (12,26 g, 84,7%) erhalten werden. ³¹P-NMR (202,4 MHz, toluene-d₈): 142,2 und 141,1 (88,1), 139,1 (11,9).

### B (4 Stunden): EtOAc/NEt₃

In einem 100 ml Schlenk wurde unter Schutzgas 10,07 g (31,0 mmol) 2,2'-Bis-(3,5-dimethylphenyl)chlorophosphit in 20 ml entgastem Ethylacetat gelöst und auf 45 °C erwärmt. In einem zweiten Schlenk (50 ml) wurden 5,54 g (15 mmol) 3,3'-Di-tert.-butyl-5,5'-dimethoxy-[1,1'-biphenyl]-2,2'diol in 26 ml Ethylacetat und 9,0 ml NEt₃ suspendiert. Anschließend wurde die Biphenol/Triethylamin-Suspension langsam innerhalb von 30 Minuten zu der Chlorophosphitlösung getropft. Die Lösung wurde vier Stunden bei 45 °C gerührt. Anschließend wurde die Lösung filtriert und der Feststoff zweimal mit ACN gewaschen. Das Zielprodukt konnte als weißer Feststoff (6,4 g, 47%) erhalten werden. ³¹P-NMR (202,4 MHz, toluene-d₈): 142,2 und 141,1 (99,3%), 139,1 (0,7%).

### C (4 Stunden): ACN/Pyridin

In einem 250 ml Schlenk wurde unter Schutzgas 10 g (31,0 mmol) 2,2'-Bis-(3,5-dimethylphenyl)chlorophosphit in 40 ml entgastem ACN gelöst und auf 45 °C erwärmt. In einem zweiten Schlenk (50 ml) wurden 5,5 g (15,0 mmol) 3,3'-Di-tert.-butyl-5,5'-dimethoxy-[1,1'-biphenyl]-2,2'diol in40 ml ACN und 8,8 ml Pyridin gelöst. Dann wurde die entstandene klare Biphenol/Pyridin-Lösung langsam innerhalb von 30 Minuten zu der Chlorophosphitlösung getropft. Nach einer Reaktionszeit von 4 Stunden wurde die Lösung filtriert und der Feststoff zweimal mit ACN gewaschen. Das Zielprodukt konnte als weißer Feststoff (8,5 g, 63%) erhalten werden. ³¹P-NMR (202,4 MHz, toluene-d₈): 142,2 und 141,1 (98,4%), 139,4 (1,6%).

### Variante 10: Tieftemperaturversuche (ACN/NEt₃)

**A:** In einem 250 ml Schlenk wurde unter Schutzgas 8,0 g (0,025 mol) 2,2'-Bis-(3,5-dimethylphenyl)chlorophosphit in 30 ml entgastem ACN gelöst und auf -40 °C gekühlt. In einem zweiten Schlenk (100 ml) wurden 4,32 g (0,012 mol) 3,3'-Di-tert.-butyl-5,5'-dimethoxy-[1,1'-biphenyl]-2,2'diol in 30 ml entgastem ACN gelöst und unter Rühren mit 8,5 ml entgastem Triethylamin versetzt. Dann wurde langsam die Biphenol/Triethylamin-Lösung zu der Chlorophosphitlösung getropft. Nach einer Nachreaktionszeit von 1h wurde die Reaktionslösung langsam über Nacht auf Raumtemperatur gebracht.
Anschließend wurde die Lösung filtriert und der Feststoff mit kaltem ACN gewaschen. Das Zielprodukt konnte als weißer Feststoff (8,9 g, 82%) erhalten werden. ³¹P-NMR (202,4 MHz, toluene-d₈): 142,5 und 140,9 (98,4%), 139,4 (1,6%).

### Variante 11: Durchführung bei verschiedenen Reaktionstemperaturen (ACN/Pyridin)

**A:** In einem 250 ml Schlenk wurde unter Schutzgas 9,4 g (28,8 mmol) 2,2'-Bis-(3,5-dimethylphenyl)chlorophosphit in 100 ml entgastem ACN gelöst. In einem zweiten Schlenk (100 ml) wurden 5,0 g (14,4 mmol) 3,3'-Di-tert.-butyl-5,5'-dimethoxy-[1,1'-biphenyl]-2,2'diol in 8,8 ml Pyridin gelöst. Dann wurde die Biphenol/Pyridin-Lösung langsam innerhalb von 1.5 Stunden zu der Chlorophosphitlösung getropft. Die Lösung wurde weitere 2 Stunden bei Raumtemperatur und anschließend über Nacht bei 60 °C gerührt.
   Anschließend wurde die Lösung filtriert und der Feststoff zweimal mit ACN gewaschen. Das Zielprodukt konnte als weißer Feststoff (9,5 g, 73%) erhalten werden. ³¹P-NMR (202,4 MHz, toluene-d₈): 142,8 und 141,2 (90%), 139,5 (10%).
**B:** In einem 250 ml Schlenk wurde unter Schutzgas 10 g (31,0 mmol) 2,2'-Bis-(3,5-dimethylphenyl)chlorophosphit in 40 ml entgastem ACN gelöst und auf 45°C erwärmt. In einem zweiten Schlenk (50 ml) wurden 5,5 g (15,0 mmol) 3,3'-Di-tert.-butyl-5,5'-dimethoxy-[1,1'-biphenyl]-2,2'diol in 40 ml ACN und 8,8 ml Pyridin gelöst. Dann wurde die entstandene klare Biphenol/Pyridin-Lösung langsam innerhalb von 30 Minuten zu der Chlorophosphitlösung getropft. Die Lösung wurde über Nacht bei 45 °C gerührt. Am nächsten Morgen wurde die Lösung filtriert und der Feststoff zweimal mit ACN gewaschen. Das Zielprodukt konnte als weißer Feststoff (9,5 g, 72%) erhalten werden. ³¹P-NMR (202,4 MHz, toluene-d₈): 142,2 und 141,1. (89,9%), 139,1 (10,1%).

### Vergleichsbeispiel Variante 12: "Eintopfsynthese"

In einem sekurierten 250 ml Schlenk wurden 8,45 g (0,0335 mol) 2,2'-Bis-(3,5-dimethylphenol) und 5,95 g (0,0166 mol) 3,3'-Di-tert.-butyl-5,5'-dimethoxy-[1,1'-biphenyl]-2,2'diol vorgelegt und unter Rühren in 50 ml getrocknetem Toluol suspendiert. Dann wurden nacheinander 7,1 g (0,051 mol) Phosphortrichlorid und 0,1 ml (0,001 mol) Pyridin bei 0 °C zu der Suspension hinzugegeben und diese Suspension wurde innerhalb von 60 Minuten auf Raumtemperatur (= RT) gebracht. Die Reaktionsmischung wurde anschließend auf 35 °C erwärmt und bei dieser Temperatur über Nacht gerührt.

Am Morgen wurden mittels ÖV bei RT das überschüssige Phosphortrichlorid und das Lösungsmittel entfernt. Im Anschluss wurde unter Rühren 25 ml entgastes ACN hinzu zugegeben und die Lösung auf 0 °C abgekühlt. In einem zweiten Schlenk (50 ml) wurden 25 ml entgastes ACN vorgelegt und unter Rühren 10,2 g = 14 ml (0,1 mol) Triethylamin hinzugegeben. Die erhaltene Lösung wurde innerhalb von 45 min. in die abgekühlte Reaktionsmixtur getropft. Dann wurde die Mischung unter Rühren über Nacht auf RT erwärmt. Am Morgen wurde der Feststoff abfiltriert, mit 2 x 25 ml entgastem ACN nachgewaschen. Das gewünschte Zielprodukt konnte in 77%iger Ausbeute (13 g) erhalten werden. ³¹P-NMR (202,4 MHz, toluene-d₈): 142.2 und 141.1 (96,4%), 139,2 (3,6%).

### Einfluss der Base /Basenmischung

### Allgemeine Synthesevorschrift

In einem 1000 ml Schlenk wurde unter Schutzgas 38,75 g (0,121 mol) 2,2'-Bis-(3,5-dimethylphenyl)chlorophosphit in 150 ml entgastem ACN gelöst und auf 45 °C erwärmt. In einem zweiten Schlenk (500 ml) wurden 20,1 g (0,056 mol) 3,3'-Di-tert.-butyl-5,5'-dimethoxy-[1,1'-biphenyl]-2,2'diol in 150 ml entgastem ACN gelöst und unter Rühren mit der entsprechenden Base (die verwendete Menge wird auf das Chlorophosphit bezogen) versetzt. Dann wurde langsam die Biphenol/Basen-Lösung zu der Chlorophosphitlösung getropft. Nach einer Nachreaktionszeit von 1h wurde die Reaktionslösung über Nacht bei 45 °C gerührt. (Anderweitige Temperaturen oder Reaktionszeiten sind den Tabellen zu entnehmen.)

Anschließend wurde die Lösung filtriert und der Feststoff mit 100 ml warmen (45 °C) ACN gewaschen. Verbindung **1a** konnte als weißer Feststoff (Ausbeute in %) erhalten werden. ³¹P-NMR (202,4 MHz, toluene-d₈): 142,5 und 140,9 (Ligand **1a** in %), 139,2 (Ligand **2a** in %).

### Syntheseroute:

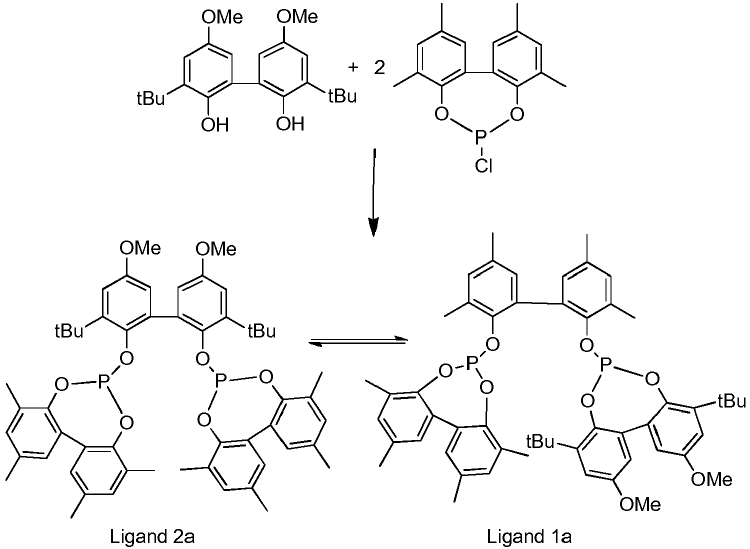

### A) Pyridin und Derivate

**Tabelle 3:**

| Äquivalent Base | Base | Anteil **1a** in [Massen-%] | Anteil **2a** in [Massen-%] | Ausbeute in [%] | |
|---|---|---|---|---|---|
| 4 | Pyridin | 72,0 | 28,0 | 81 | * |
| 4 | Pyridin | 74,0 | 26,0 | 81 | |
| 3 | Pyridin | 80,6 | 19,4 | 80 | |
| 2,5 | Pyridin | 81,9 | 18,1 | 78 | |
| 2 | Pyridin | 84,2 | 15,8 | 78 | |
| 1.7 | Pyridin | 84,2 | 15,8 | 88 | |
| 1,5 | Pyridin | 86,3 | 13,7 | 79 | |
| 1,5 | Pyridin | 84,5 | 15,5 | 82 | ** |
| 2,5 | Pyridin | 81,8 | 18,2 | 78 | *** |
| 2,5 | Pyridin | 86,8 | 13,2 | 81 | **** |
| 2,5 | DMAP | 46,6 | 53,4 | 51 | |
| 2 | DMAP | 42,7 | 57,3 | 50 | # |
| 2 | DMAP | 47,8 | 52,2 | 89 | ## |
| 2 | DMAP | 65,1 | 34,9 | 90 | ### |
| 2 | 2-Picolin | 76,0 | 24,0 | 67 | |

| | | | | | |
|---|---|---|---|---|---|
| DMAP = Dimethylaminopyridin *: Versuch bei 0°C **: Versuch bei 50°C ***: verlängerte Reaktionszeit (5 Tage) ****: sofortige Zugabe anstelle des langsamen Zutropfens #: Reaktion bei 0°C ##: Reaktion bei 3-7°C ###: Reaktion bei 45°C | | | | | |

Wie in Tabelle 3 deutlich erkennbar, ist es möglich, die Isomerenverteilung der beiden Konstitutionsisomere (1 a) und (2a) durch die Wahl der Base bzw. der entsprechenden Basenmenge zu steuern. So ist es beispielsweise möglich eine 1:1 Mischung der beiden Isomeren (1a) und (2a) durch Verwendung von DMAP als Base bei niedrigeren Temperaturen zu erhalten.

### B) verschiedene Alkylamine

**Tabelle 4:**

| Äquivalent Base | Base | Anteil 1a in [Massen-%] | Anteil 2a in [Massen-%] | Ausbeute in [%] |
|---|---|---|---|---|
| 2,2 | NEt₃ | 95,4 | 4,6 | 86 |
| 2,3 | DMAB | 97,4 | 2,6 | 86 |
| 2,2 | Tributylamin | 94,4 | 5,6 | 90 |
| 2 | Tripentylamin | 96,0 | 4,0 | n.b. |
| 2 | Trihexylamin | 97,8 | 2,2 | 94 |

| | | | | |
|---|---|---|---|---|
| NEt₃: Triethylamin DMAB: Dimethylaminobutan n.b.: nicht bestimmt | | | | |

Wie in Tabelle 4 deutlich erkennbar, ist es möglich, durch die Wahl von Trialkylaminen als Base eine Isomerenmischung zu erhalten, in der das unsymmetrische Isomer (1 a) mit einer Reinheit von > 90% als Hauptkomponente vorliegt und das symmetrische Isomer (2a) die entsprechende Nebenkomponente darstellt.

### C) verschiedene Basenmischungen

**Tabelle 5:**

| Basen | Verhältnis | Anteil **1a** in [Massen-%] | Anteil **2a** in [Massen-%] | Ausbeute in [%] |
|---|---|---|---|---|
| Pyr/NEt₃ | 4:1 | 78,2 | 21,8 | 56 |
| Pyr/NEt₃ | 4:0,5 | 59,6 | 40,4 | 87 |
| Pyr/NEt₃ | 4:0,25 | 59,7 | 40,3 | 80 |
| Pyr/NEt₃ | 3:0,5 | 62,4 | 37,6 | 81 |
| Pyr/NEt₃ | 2:0,5 | 69,1 | 30,9 | 84 |
| Pyr/NBu₃ | 2:0,5 | 72,4 | 27,6 | 78 |
| Pyr/NBu₃ | 2:0,25 | 47,9 | 52,1 | 81 |
| Pyr/NBu₃ | 2:2 | 91,8 | 8,2 | 83 |
| Pyr/NBu₃ | 2,5:0,2 | 81,0 | 19,0 | 80 |
| Pyr/NBu₃ | 2,5:2 | 92,6 | 7,4 | 69 |

| | | | | |
|---|---|---|---|---|
| NEt₃: Triethylamin NBu3: Tributylamin Pyr: Pyridin | | | | |

Wie in Tabelle 5 deutlich erkennbar, ist es möglich, die Isomerenverteilung der beiden Konstitutionsisomere (1a) und (2a) durch den Einsatz von Basenmischungen und deren entsprechenden Basenmenge zu steuern.

Es ist somit möglich, die Isomerenverteilung der beiden Konstitutionsisomeren (1 a) und (2a) durch die Wahl der verwendeten Base bzw. Basenmischung so zu beeinflussen, sodass ein Isomer als Hauptkomponente vorliegt. Durch die Wahl von Trialkylaminen als Base ist es möglich eine Isomerenmischung zu erhalten, in der das unsymmetrische Isomer (1 a) mit einer Reinheit von > 90% als Hauptkomponente vorliegt und das symmetrische Isomer (2a) die entsprechende Nebenkomponente darstellt. Da diese Mischung auch in der Hydroformylierung eine sehr gute Gesamtperformance zeigt, kann auf weitere Aufreinigungsschritte verzichtet werden.

### Erfindungsgemäße Synthese des Liganden (3Ia) - Allgemeine Reaktionsgleichung

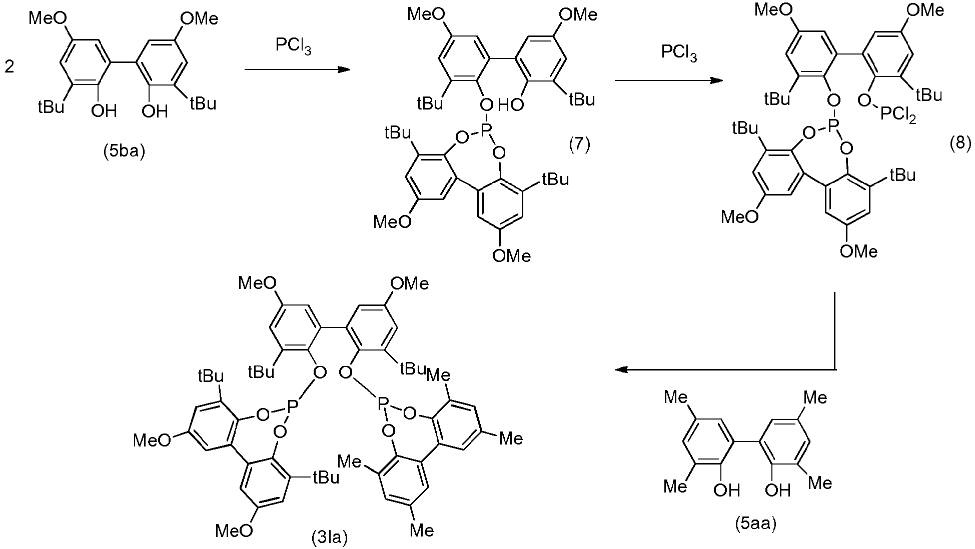

### Synthese des Phosphits (7)

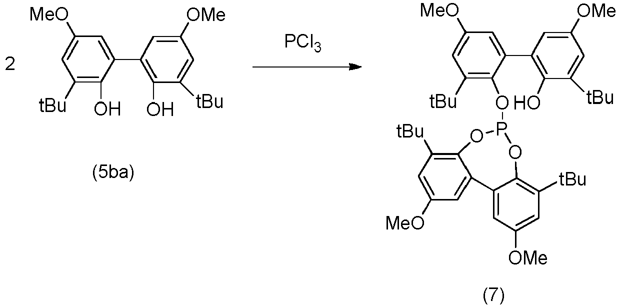

In einem sekurierten 1000 ml Schlenk wird 400 ml getrocknetes Toluol vorgelegt und mittels Spritze 8.9 ml (0.1 mol) Phosphortrichlorid hinzugegeben und auf 0 °C abgekühlt.

In einem 500 ml Schlenk werden 71.6 g (0.2 mol) 3,3'-Ditert.-butyl-2,2'-dihydroxy-5,5'-dimethoxybiphenyl abgewogen und in 325 ml getrocknetem Toluol und 49 ml (0.35 mol) getrocknetem Triethylamin gelöst.

Nun wird die Biphenol/Et₃N/Toluol-Suspension innerhalb von 2.5 h zur 0 °C gekühlten PCl₃/Toluol-Lösung getropft und über Nacht bei RT reagieren gelassen.

Am nächsten Morgen wurde der entstandene Feststoff filtriert, mehrmals mit getrocknetem Toluol nachgewaschen und das Filtrat bis zur Trockene eingeengt. Um einen weißen Feststoff zu erhalten, wurden weitere Male mit ACN nachgewaschen. Das Zielprodukt konnte so in 79.5%iger Ausbeute (59,1 g) erhalten werden.

### Synthese des Diorganophosphitdichlorophosphits (8)

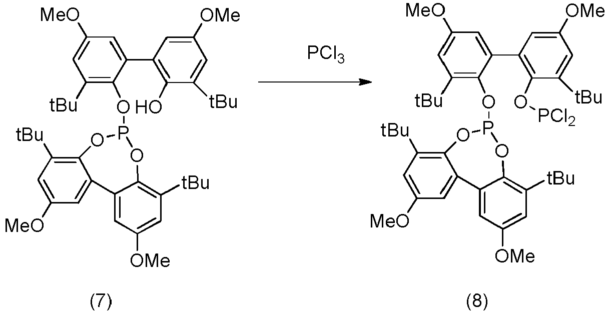

In einem sekurierten 250 ml Schlenk wurden 42 g (0,056 mol) des Phosphits (7) abgewogen und unter Rühren 275 ml getrocknetes Toluol und 17 ml (0,168 mol) getrocknetes Triethylamin hinzugegeben.

In einem zweiten 1000 ml Schlenk wurden zunächst 200 ml getrocknetes Toluol vorgelegt und anschließend 14,76 ml (0,169 mol) Phosphortrichlorid zugegeben. Anschließend wurde unter kräftigem Rühren tropfenweise zu der Phosphortrichlorid/Toluol-Lösung die zuvor hergestellte Phosphit/Amin/Toluol-Lösung innerhalb von 30 Minuten bei RT getropft. Nach vollständiger Zugabe wurde die Reaktionsmischung für 6 h auf 80 °C erwärmt und über Nacht auf RT kommen lassen.

Am nächsten Morgen wurde filtriert, mit 50 ml getrocknetem Toluol nachgewaschen und das Filtrat bis zur Trockne eingeengt. Das Produkt konnte in 89%iger Ausbeute (45,6 g) erhalten werden.

### Erfindungsgemäße Synthese des Liganden (31a)

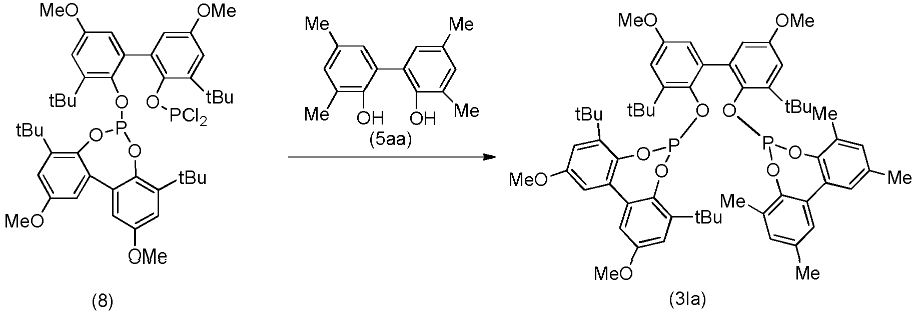

In der Glove-Box wurde in einem sekurierten 100 ml Schlenk 3,08 g (0,0036 mol) Diorganophosphitdichlorophosphit (8) eingewogen und anschließend in 35 ml getrocknetem Toluol gelöst.

In einem zweiten sekurierten 250 ml Schlenk wurden 0,872 g (0,0036 mol) 2,2'-Bis(3,5-dimethylphenol) und 1,09 g (0,01 mol) getrocknetes Triethylamin in 35 ml Toluol gelöst.

Dann wurde zur Biphenyl-Triethylamin-Lösung langsam und stetig unter kräftigem Rühren bei RT das Diorganophosphitdichlorophosphit (8) hinzugetropft. Anschließend wurde die Reaktionsmischung über Nacht gerührt.

Zur Aufarbeitung wurde der entstandene Feststoff am nächsten Morgen filtriert und zweimal mit 5 ml getrocknetem Toluol nachgewaschen. Das erhaltene Filtrat wurde dann bis zur Trockne eingeengt. Das Zielprodukt konnte als weißer Feststoff (2,59 g; 71%) erhalten werden.

### Erfindungsgemäße Synthese des Liganden (4IIa) - Allgemeine Reaktionsgleichung

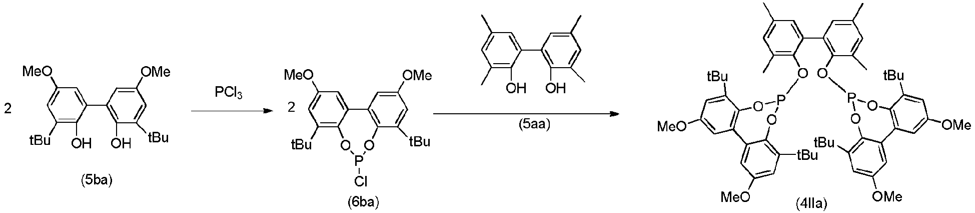

### Herstellung des 3,3'-tert.-butyl-2,2'-dihydroxy-5,5'-dimethoxybiphenyl-chlorophosphits (6ba)

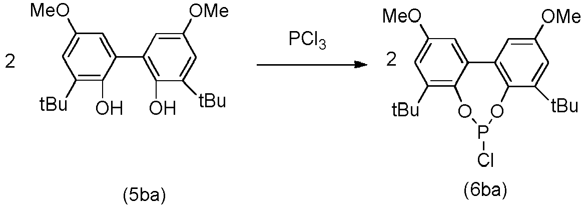

In einem 500 ml Schlenk wurden 35,8 g (0,1 mol) 3,3'-tert.-butyl-2,2'-dihydroxy-5,5'-dimethoxybiphenol eingewogen und das Biphenol in 42,3 ml (0,3 mol) entgastem Triethylamin und 250 ml getr. Toluol gelöst.

In einem zweiten sekurierten 1 L Schlenk wurden 8,8 ml (0,1 mol) PCl₃ in 300 ml getrocknetes Toluol vorgelegt und auf 0 °C abgekühlt. Zu dieser PCl₃/Toluol-Lsg. wurde nun vorsichtig unter kräftigem Rühren die zuvor hergestellte Phenol/Amin-Lösung hinzugetropft.

Nach dem Zutropfen wurde die Lösung über Nacht auf RT erwärmt. Am nächsten Morgen wurde der entstandene Feststoff abfiltriert und das Lösungsmittel bis zur Trockene eingeengt. Das Produkt konnte als honigartiger Rückstand in 56%iger Ausbeute gewonnen werden (27,5 g).

### Erfindungsgemäße Synthese des Liganden (411a)

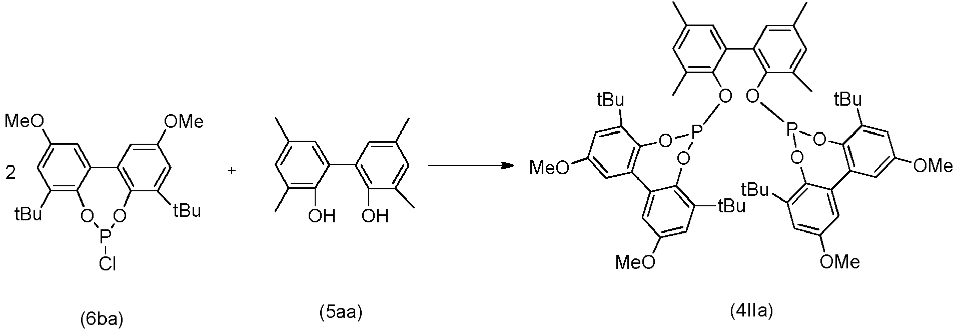

In einem sekurierten 250 ml Schlenk wurden 9,79 g (0,022 mol) Chlorophosphit (6ba) eingewogen und anschließend in 75 ml getrocknetem Toluol gelöst.

Im einem weiteren sekurierten 100 ml Schlenk wurden 2,66 g (0,011 mol) 2,2'-Bis(3,5-dimetylphenol) und 2,46 g (0,022 mol) Kalium-tert.-butylat eingewogen und in 70 ml getrocknetem Toluol unter Rühren zugegeben.

Zu der vorgelegten Chlorophosphitlösung wurde bei RT, langsam und stetig die Biphenol/Kalium-tert.-butylat-Mischung unter Rühren zugetropft. Anschließend wurde über Celite abfiltriert. Die Lösung wurde eingeengt und der verbleibende Rückstand mit 50 ml getrocknetem Acetonitril gewaschen. Das Zielprodukt konnten in 25.5%iger Ausbeute (2,76 g) erhalten werden.

### Arbeitsvorschrift für die Hydroformylierungsexperimente

### Versuchsbeschreibung - allgemein

Die Versuche wurden in 100 ml-Autoklaven der Fa. Parr Instrument durchgeführt. Die Autoklaven sind mit einer elektrischen Beheizung ausgerüstet. Die Druckkonstanthaltung erfolgt über Massedurchflußmesser und Druckregler. Während der Versuchszeit kann über eine Spritzenpumpe eine genau definierte Eduktmenge unter Reaktionsbedingungen eingespritzt werden. Über Kapillarleitungen und HPLC-Ventile können während der Versuchszeit Proben gezogen und sowohl über GC- als auch über LC-MS-Analytik untersucht werden.

### Erfindungsgemäße Ergebnisse der Testung der verschiedenen

### Verbindungenmischungen der Liganden (1Ia) und (2IIa) in der Hydroformylierung^{[a]}:

**Tabelle 6:**

| **Nr** | **Ligand** | **Gehalt an Ligand in [%]** | **Pentanalselektivität in [%]^{[b]}** | **Ausbeute In [%]^{[b]}** |
|---|---|---|---|---|
| 1 | Ligand (1Ia) | 100 | 94,0 | 92,9 |
| 2* | Ligand (1Ia) + Ligand (2IIa) | 99.3 + 0.7 | 93,9 | 91,0 |
| 3* | Ligand (1Ia) + Ligand (2IIa) | 91.9 + 8.1 | 93,7 | 93,1 |
| 4* | Ligand (1Ia) + Ligand (2IIa) | 90.3 + 9.7 | 93,8 | 92,6 |
| 5* | Ligand (1Ia) + Ligand (2IIa) | 74 + 26 | 93,7 | 92,7 |
| 6* | Ligand (1Ia) + Ligand (2IIa) | 80 + 20 | 92,5 | 92,5 |
| 7* | Ligand (1Ia) + Ligand (2IIa) | 98.7 + 1.3 | 87,9 | 78,7 |

| | | | | |
|---|---|---|---|---|
| * erfindungsgemäß | | | | |

[a] Bedingungen: cis-2-Buten, Rh(acac)(CO)₂ ([Rh]= 95 ppm), L/Rh = 6:1, 40 ml Toluol, Verbindung 11, 120°C, 20 bar CO/H₂ (1:1), 1,2,4,5-Tetra-isopropyl-benzol als interner GC-Standard. [b] GC-Analyse mit 1,2,4,5-Tetra-isopropyl-benzol als interner GC-Standard. # weitere Nebenkomponenten u.a. nicht umgesetztes Chlorophosphit in größeren Mengen enthalten. Die gewünschte Zusammensetzung aus den beiden Liganden (1Ia) und (2IIa) ist nur in einer Reinheit von 30% in einem Gemisch mit anderen Komponenten/Verunreinigungen enthalten.

Bei einem Vergleich der verschiedenen Ligandenmischungen aus den Liganden (1Ia) und (2IIa) (Tabelle 6, Einträge 2-6) mit dem Hydroformylierungsergebnis des reinen Liganden (1Ia) (Tabelle 6, Eintrag 1) zeigt sich, dass die Mischungen sehr gute Pentanalselektivitäten und Ausbeuten aufweisen. Auch bei der Verwendung einer Ligandenmischung, in der der Ligand (1Ia) nur in einer Reinheit von ca. 30% enthalten ist (Tabelle 6, Eintrag 7) konnte immer noch eine sehr gute Ausbeute und Selektivität generiert werden. Durch die Verwendung dieses Gemisches von Verbindungen Bisphosphiten, bestehend aus Ligand (1Ia) und (2IIa), konnte die technische Aufgabe somit vollends erfüllt werden und die entsprechenden Aldehyde in guten bis sehr guten Ausbeuten und Selektivitäten erhalten werden.

Es konnte somit gezeigt werden, dass in Hydroformylierungsreaktionen auch Ligandenmischungen eingesetzt werden können.

### Erfindungsgemäße Ergebnisse der Testung der verschiedenen

### Verbindungenmischungen der Liganden (1Ia), (3Ia) und (4IIa) in der Hydroformylierung^{[a]}:

**Tabelle 7:**

| **Nr** | **Liganden** | **Gehalt an Liganden** | **Verhältnis der Liganden in [%]^{[e]}** | **Pentanalselektivität in mol [%]^{[b]}** | **Ausbeute In [%]^{[b]}** |
|---|---|---|---|---|---|
| 1 | Ligand (3Ia) | 100% | L3Ia: 100% | 53,2 | 76,2 |
| 2 | Ligand (4IIa) | 100% | L4Ia: 100% | 61,8[d] | 76,2 |
| 3* | Ligand (3Ia) + Ligand (4IIa) | L3Ia:L4IIa:Rh 3,7:0,41:1 | L3Ia: 90% + L4IIa: 10% | 43,9 | 77,2 |
| 4* | Ligand (3Ia) + Ligand (4IIa) | L3Ia:L4IIa:Rh 2,96:1,07:1 | L3Ia: 73% + L4IIa: 27% | 46,0 | 72,4 |
| 5* | Ligand (3Ia) + Ligand (4IIa) | L3Ia:L4IIa:Rh 1,8:1,8:1 | L3Ia: 50% + L4IIa: 50% | 42,8 | 74,4 |
| 6* | Ligand (3Ia) + Ligand (4IIa) | L3Ia:L4IIa:Rh 1,1:3,1:1 | L3Ia: 26% + L4IIa: 74% | 37,1 | 72,2 |
| 7* | Ligand (3Ia) + Ligand (4IIa) | L3Ia:L4IIa:Rh 0,41:3,55:1 | L3Ia: 10% + L4IIa 90% | 32,7 | 72,4 |

| | | | | | |
|---|---|---|---|---|---|
| *erfindungsgemäß | | | | | |

[a] Bedingungen: cis-2-Buten, Rh(acac)(CO)₂, Toluol, Verbindung (11), 120°C, 20 bar CO/H₂ (1:1), 1,2,4,5-Tetra-isopropyl-benzol oder Mesitylen als internen GC-Standard. [b] GC-Analyse mit 1,2,4,5-Tetra-isopropyl-benzol oder Mesitylen als internen GC-Standard. [c] Pentanalselektivität und Ausbeute in [%]. [d] Aldehydausbeute in [%]. [e] Verhältnis der beiden Liganden zueinander auf 100% normiert.

Die reinen Liganden (3Ia) und (4IIa) (Tabelle 7, Einträge 1 und 2) zeigen gute Pentanalselektivitäten und Ausbeuten. Neben den reinen Liganden ist es aber auch möglich verschiedenen Ligandenmischungen aus den Liganden (3Ia) und (4IIa) (Tabelle 7, Einträge 3-7) einzusetzen.

### Versuchsbeschreibung - Langzeitversuch

Die Versuche wurden in 100 ml-Autoklaven der Fa. Parr Instrument durchgeführt. Die Autoklaven sind mit einer elektrischen Beheizung ausgerüstet. Die Druckkonstanthaltung erfolgt über Massedurchflußmesser und Druckregler. Während der Versuchszeit kann über eine Spritzenpumpe eine genau definierte Eduktmenge unter Reaktionsbedingungen eingespritzt werden. Über Kapillarleitungen und HPLC-Ventile können während der Versuchszeit Proben gezogen und sowohl über GC- als auch über LC-MS-Analytik untersucht werden.

Der Rh-Precursor (Rh(acac)(CO)₂) und der Ligand bzw. die Ligandenmischungen werden in 40 ml Isononylbenzoat im Autoklaven vorgelegt. Die Rh-Konzentration beträgt 100 ppm bezogen auf die gesamte eingesetzte Reaktionsmasse. Der Ligandüberschuß beträgt molar 4:1 bezogen auf Rhodium.
Als Stabilisator wird im Verhältnis 2:1 zum Liganden die Verbindung (11) als Amin zugegeben. Als GC-Standard werden 0,5 g 1,2,4,5-Tetraisopropylbenzol hinzugegeben. Reaktionstemperatur ist 120 °C. Der Reaktionsdruck beträgt 20 bar Synthesegas (H₂:CO=50:50 Vol%).

Als Olefin wurden mit der Spritzenpumpe in Abständen von ca. 1 Tag jeweils 4 ml cis-2-Buten zudosiert. GC-Proben wurden nach 1, 2, 4 Stunden und vor der nächsten Dosierung gezogen. Es wurden folgende Liganden hinsichtlich ihrer Stabilität untersucht:

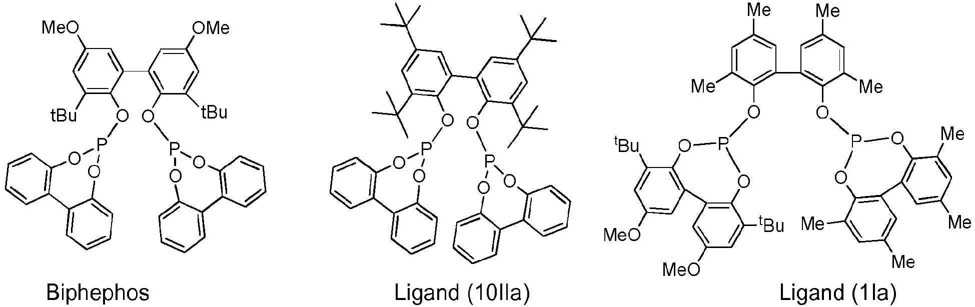

Ferner wurden Mischungen untersucht: Ligand (1Ia) und Ligand (2IIa) (³¹P-NMR: L1la=91% und L2IIa=9%) 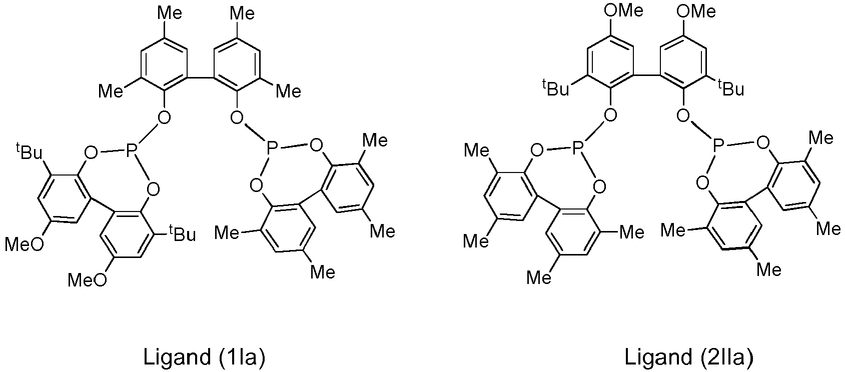 sowie eine Mischung aus Ligand (10IIa) und Ligand (9Ia) (³¹P-NMR: L10IIa=75% und L9Ia=25%)

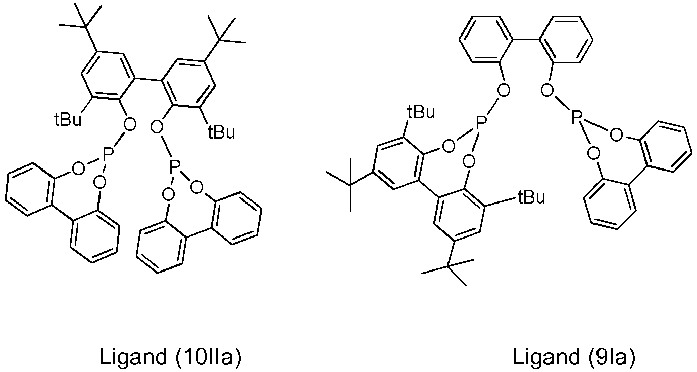

### Ergebnisse - Langzeitversuche

Die relativen Aktivitäten werden durch das Verhältnis von k 1.Ordnung zu k0, d.h. dem k-Wert zum Zeitpunkt 0 der Reaktion (Reaktionsstart), bestimmt und beschreiben die relative Aktivitätsabnahme während der Versuchslaufzeit.

Die k-Werte 1. Ordnung erhält man aus einer Auftragung von (-ln(1-Umsatz)) gegen die Zeit.

**Tabelle 8:**

| **Lfd. Nr.** | **Ligand** | **Dosierung bei Laufzeit (h)** | **k 1. Ordnung (min^-1)** | **k/k0 rel. Aktivität** | **n**/**i-Selektivitäten** |
|---|---|---|---|---|---|
| 1 | Biphephos | 0 | 1,39E-02 | 1 | 21 |
| 2 | Biphephos | 20,5 | 4,45E-03 | 0,32 | 21 |
| 3 | Biphephos | 44,3 | 2,91 E-03 | 0,209 | 20 |
| 4 | Biphephos | 66,6 | 1,72E-03 | 0,124 | 20 |
| 5 | Ligand (10IIa) + Ligand (9Ia) | 0 | 1,36E-02 | 1 | 3,1 |
| 6 | Ligand (10IIa) + Ligand (9Ia) | 20,5 | 5,32E-03 | 0,391 | 2,4 |
| 7 | Ligand (10IIa) + Ligand (9Ia) | 44,3 | 4,80E-03 | 0,353 | 1,8 |
| 8 | Ligand (1Ia) | 0 | 7,74E-03 | 1 | 17 |
| 9 | Ligand (1Ia) | 20,8 | 5,10E-03 | 0,659 | 16 |
| 10 | Ligand (1Ia) | 44,8 | 3,19E-03 | 0,412 | 15 |
| 11 | Ligand (1Ia) | 117,8 | 2,99E-03 | 0,386 | 14 |
| 12* | Ligand (1 la) + Ligand (2IIa) | 0 | 1,09E-02 | 1 | 14 |
| 13* | Ligand (1Ia) + Ligand (2IIa) | 20,8 | 5,65E-03 | 0,518 | 14 |
| 14* | Ligand (1Ia) + Ligand (2IIa) | 44,8 | 4,13E-03 | 0,379 | 13 |
| 15* | Ligand (1Ia) + Ligand (2IIa) | 117,8 | 3,35E-03 | 0,307 | 13 |
| 16 | Ligand (10IIa) | 0 | 1,72E-02 | 1 | 14 |
| 17 | Ligand (10IIa) | 22,4 | 9,00E-03 | 0,523 | 13 |
| 18 | Ligand (10IIa) | 44,7 | 5,39E-03 | 0,313 | 13 |
| 19 | Ligand (10IIa) | 68,3 | 3,31E-03 | 0,192 | 13 |

| | | | | | |
|---|---|---|---|---|---|
| * erfindungsgemäß | | | | | |

Der Aktivitätsabfall des Katalysators mit den Liganden Biphephos und Ligand (10IIa) ist (Tabelle 8; Einträge 1-4, 16-19) deutlich stärker als mit dem Liganden (1Ia) (Tabelle 8; Einträge 8-11). Bemerkenswert ist, dass die relative Aktivität des Liganden (1Ia) nach annähernd der doppelten Reaktionszeit (Tabelle 8; Eintrag 11) immer noch mehr als doppelt so hoch ist wie bei den anderen beiden Liganden nach der halben Reaktionszeit (Tabelle 5; Einträge 4 und 19). Weiterhin ist das n/i-Verhältnis beim Katalysator mit dem Liganden (10IIa) weiterhin sehr hoch. Vergleicht man das Gemisch der Liganden (1Ia) + (2IIa) mit dem reinen Liganden (1Ia) (Tabelle 8; Einträge 8-11, 12-15) so zeigt das Gemisch nach 117 Stunden Laufzeit eine vergleichbare Aktivität und Selektivität wie der reine Ligand (1Ia). Das Gemisch aus den Liganden (10IIa) + (9Ia) zeigt von Anfang an eine deutlich schlechtere Selektivität als der reine Ligand (10IIa) wie auch das Gemisch aus Ligand (1Ia) + (2IIa) (Tabelle 8; Einträge 5-7, 12-15 und 16-19).

Die Zugabe des unsymmetrischen Liganden (9Ia) zum symmetrischen Liganden (10IIa) führt zu einem drastischen Selektivitätseinbruch (Tabelle 8; Einträge 5-7). Dies entspricht den Ergebnissen aus dem SdT (siehe in Rhodium-catalyzed Hydroformylation, ed. by P.W.N.M. van Leeuwen et C. Claver, Kluwer Academic Publishers 2006, AA Dordrecht, NL, Seite 45- 46). Im völligen Gegensatz dazu zeichnet sich der unsymmetrische Ligand (1Ia), sowohl als Reinstoff als auch in der Mischung mit dem Liganden (2IIa) (Tabelle 8; Einträge 8-11, 12-15) völlig überraschend durch exzellente Standzeiten als auch sehr gute Selektivitäten aus. Ferner konnte gezeigt werden, dass somit auch Mischungen von konstitutionsisomeren Liganden
(1Ia) und (2IIa) ohne zusätzliche aufwendige Reinigungsprozesse direkt aus der Synthese eingesetzt werden.

### Erfindungsgemäße Ergebnisse - Substratvariation

Für die nachfolgenden Versuche wurde folgende Mischung untersucht: Ligand (1Ia) + Ligand (2IIa) (³¹P-NMR: LI1a=91% + LI2a=9%).

### Beispiel 1

In einem 100 ml-Autoklaven der Fa. Parr Instruments wurde bei 120 °C und 30 bar 4.8 g Propen hydroformyliert. Als Precursor wurden 0.005 g Rh(acac)(CO)₂ in 43.08 g Toluol vorgelegt. Als Ligand wurden 0.0708 g der oben beschriebenen Ligandenmischung (³¹P-NMR: LI1a=91% + LI2a=9%) in der Katalysatoransatzlösung eingesetzt. Als organisches Amin wurden 0.0401 g der Verbindung (11) und 0.5033 g TIPB als GC-Standard zugefügt. Das Edukt wurde nach Erreichen der vorgesehenen Reaktionstemperatur zudosiert.

Während der Reaktion wurde der Druck über eine Synthesegasregelung mit Massedurchflussmesser konstant gehalten. Proben wurden aus der Reaktionsmischung nach 20 Stunden gezogen. Es wurden 88,4 mol-% Butanal, 6,48 mol-% 2-Methylpropanal und 2,79 mol-% Propan gebildet. Die Regioselektivität zu n-Butanal beträgt 93.2 %.

### Beispiel 2

In einem 100 ml-Autoklaven der Fa. Parr Instruments wurde bei 120 °C und 20 bar 6.7 g cis-2-Buten hydroformyliert. Als Precursor wurden 0.0053 g Rh(acac)(CO)₂ in 43.48 g Toluol vorgelegt. Als Ligand wurden 0.0671 g der oben beschriebenen Ligandenmischung (³¹P-NMR: LI1a=91% + LI2a=9%) in der Katalysatoransatzlösung eingesetzt. Als organisches Amin wurden 0.0381 g der Verbindung (11) und 0.5099 g TIPB als GC-Standard zugefügt. Das Edukt wurde nach Erreichen der vorgesehenen Reaktionstemperatur zudosiert.

Während der Reaktion wurde der Druck über eine Synthesegasregelung mit Massedurchflussmesser konstant gehalten. Proben wurden aus der Reaktionsmischung nach 20 Stunden gezogen. Es wurden 84.6 mol-% Pentanal, 5.70 mol-% 2-Methylbutanal und 3.43 mol-% n-Butan gebildet. Die Regioselektivität zu n-Pentanal beträgt 93.7 %.

### Beispiel 3

In einem 100 ml-Autoklaven der Fa. Parr Instruments wurde bei 120 °C und 20 bar 6.7 g 1-Buten hydroformyliert. Als Precursor wurden 0.0052 g Rh(acac)(CO)₂ in 43.08 g Toluol vorgelegt. Als Ligand wurden 0.0694 g der oben beschriebenen Ligandenmischung (³¹P-NMR: LI1a=91% + LI2a=9%) in der Katalysatoransatzlösung eingesetzt. Als organisches Amin wurden 0.0378 g der Verbindung (11) und 0.5052 g TIPB als GC-Standard zugefügt. Das Edukt wurde nach Erreichen der vorgesehenen Reaktionstemperatur zudosiert.

Während der Reaktion wurde der Druck über eine Synthesegasregelung mit Massedurchflussmesser konstant gehalten. Proben wurden aus der Reaktionsmischung nach 20 Stunden gezogen. Es wurden 86.5 mol-% Pentanal, 5.08 mol-% 2-Methylbutanal und 3.23 mol-% n-Butan gebildet. Die Regioselektivität zu n-Pentanal beträgt 98.9 %.

### Beispiel 4

In einem 100 ml-Autoklaven der Fa. Parr Instruments wurde bei 120 °C und 20 bar 6.7 g Isobuten hydroformyliert. Als Precursor wurden 0.0051 g Rh(acac)(CO)₂ in 42.1 g Toluol vorgelegt. Als Ligand wurden 0.0678 g der oben beschriebenen Ligandenmischung (³¹P-NMR: LI1a=91% + LI2a=9%) in der Katalysatoransatzlösung eingesetzt. Als organisches Amin wurden 0.0369 g der Verbindung (11) und 0.4937 g TIPB als GC-Standard zugefügt. Das Edukt wurde nach Erreichen der vorgesehenen Reaktionstemperatur zudosiert.

Während der Reaktion wurde der Druck über eine Synthesegasregelung mit Massedurchflussmesser konstant gehalten. Proben wurden aus der Reaktionsmischung nach 20 Stunden gezogen. Es wurden 64.0 mol-% 3-Methylbutanal, 0.07 mol-% Pivalinaldehyd und 2.92 mol-% iso-Butan gebildet.

### Beispiel 5

In einem 100 ml-Autoklaven der Fa. Parr Instruments wurde bei 120 °C und 20 bar 7.4 g eines C-4-Gemisches mit folgender Zusammensetzung: 2.9 mol-% Isobutan, 9,9 mol-% n-Butan, 28.7 mol-% 1-Buten, 43.5 mol-% Isobuten, 14,6 mol % 2-Butene und 0.2 mol % 1,3-Butadien. hydroformyliert. Als Precursor wurden 0.0048 g Rh(acac)(CO)₂ in 41.49 g Toluol vorgelegt. Als Ligand wurden 0.0681 g der oben beschriebenen Ligandenmischung (³¹P-NMR: LI1a=91% + LI2a=9%) in der Katalysatoransatzlösung eingesetzt. Als organisches Amin wurden 0.0367 g der Verbindung (11) und 0.5027 g TIPB als GC-Standard zugefügt. Das Edukt wurde nach Erreichen der vorgesehenen Reaktionstemperatur zudosiert.

Während der Reaktion wurde der Druck über eine Synthesegasregelung mit Massedurchflussmesser konstant gehalten. Proben wurden aus der Reaktionsmischung nach 20 Stunden gezogen. Der Austrag enthält 32.7 % 3-Methylbutanal (Umsatz Isobuten 75.2 mol -%), 39.44 mol-% n-Pentanal und 2.18 mol-% 2-Methylbutanal (Umsatz Butene 78.1 mol-%, Regioselektivität zu n-Pentanal 94.8 %). Als Hydrierprodukte werden im Austrag 4.13 mol-% Isobutan und 9.95 mol % n-Butan gefunden.

### Beispiel 6

In einem 100 ml-Autoklaven der Fa. Parr Instruments wurde bei 120 °C und 20 bar 7.0 g eines C-4-Gemisches mit folgender Zusammensetzung: 5.9 mol-% Isobutan, 15.6 mol-% n-Butan, 52.9 mol-% 1-Buten, 0.1 mol-% Isobuten, 24.8 mol % 2-Butene und 0.5 mol % 1,3-Butadien hydroformyliert. Als Precursor wurden 0.0054 g Rh(acac)(CO)₂ in 46.93 g Toluol vorgelegt. Als Ligand wurden 0.0755 g der oben beschriebenen Ligandenmischung (³¹P-NMR: LI1a=91% + LI2a=9%) in der Katalysatoransatzlösung eingesetzt. Als organisches Amin wurden 0.0412 g der Verbindung (11) und 0.5467 g TIPB als GC-Standard zugefügt. Das Edukt wurde nach Erreichen der vorgesehenen Reaktionstemperatur zudosiert.

Während der Reaktion wurde der Druck über eine Synthesegasregelung mit Massedurchflussmesser konstant gehalten. Proben wurden aus der Reaktionsmischung nach 20 Stunden gezogen. Der Austrag enthält 0.17 mol-% 3-Methylbutanal, 70.31 mol-% n-Pentanal und 4.20 mol-% 2-Methylbutanal (Umsatz Butene 93.4 mol-%, Regioselektivität zu n-Pentanal 94.4 %). Als Hydrierprodukte werden im Austrag 5.52 mol-% Isobutan und 18.1 mol % n-Butan gefunden.

### Beispiel 7

In einem 100 ml-Autoklaven der Fa. Parr Instruments wurde bei 120 °C und 20 bar 5.0 g eines C-4-Gemisches mit folgender Zusammensetzung: 5.9 mol-% Isobutan, 22.0 mol-% n-Butan, 45.5 mol-% 1-Buten, 2.1 mol-% Isobuten, 17.1 mol % 2-Butene und 0.2 mol % 1,3-Butadien hydroformyliert. Als Precursor wurden 0.0044 g Rh(acac)(CO)₂ in 37.96 g Toluol vorgelegt. Als Ligand wurden 0.0611 g der oben beschriebenen Ligandenmischung (³¹P-NMR: LI1a=91% + LI2a=9%) in der Katalysatoransatzlösung eingesetzt. Als organisches Amin wurden 0.0333 g der Verbindung (11) und 0.4422 g TIPB als GC-Standard zugefügt. Das Edukt wurde nach Erreichen der vorgesehenen Reaktionstemperatur zudosiert.

Während der Reaktion wurde der Druck über eine Synthesegasregelung mit Massedurchflussmesser konstant gehalten. Proben wurden aus der Reaktionsmischung nach 20 Stunden gezogen. Der Austrag enthält 1.52 mol-% 3-Methylbutanal (Umsatz Isobuten 72.1 mol-%), 63.2 mol-% n-Pentanal und 3.13 mol-% 2-Methylbutanal (Umsatz Butene 95.6 mol-%, Regioselektivität zu n-Pentanal 95.3 %). Als Hydrierprodukte werden im Austrag 5.41 mol-% Isobutan und 23.89 mol % n-Butan gefunden.

### Beispiel 8

In einem 100 ml-Autoklaven der Fa. Parr Instruments wurde bei 120 °C und 20 bar 6.4 g eines C-4-Gemisches mit folgender Zusammensetzung: 3.4 mol-% Isobutan, 13.0 mol-% n-Butan, 47.3 mol-% 1-Buten, 13.9 mol-% Isobuten, 21.6 mol % 2-Butene und 0.4 mol % 1,3-Butadien hydroformyliert. Als Precursor wurden 0.0052 g Rh(acac)(CO)₂ in 44.95 g Toluol vorgelegt. Als Ligand wurden 0.0704 g der oben beschriebenen Ligandenmischung ((³¹P-NMR: LI1a=91% + LI2a=9%) in der Katalysatoransatzlösung eingesetzt. Als organisches Amin wurden 0.0387 g der Verbindung (11) und 0.5318 g TIPB als GC-Standard zugefügt. Das Edukt wurde nach Erreichen der vorgesehenen Reaktionstemperatur zudosiert.

Während der Reaktion wurde der Druck über eine Synthesegasregelung mit Massedurchflussmesser konstant gehalten. Proben wurden aus der Reaktionsmischung nach 20 Stunden gezogen. Der Austrag enthält 9.93 mol-% 3-Methylbutanal (Umsatz Isobuten 71.7 mol-%), 62.6 mol-% n-Pentanal und 2.98 mol-% 2-Methylbutanal (Umsatz Butene 95.6 mol-%, Regioselektivität zu n-Pentanal 95.5 %). Als Hydrierprodukte werden im Austrag 3.59 mol-% Isobutan und 15.41 mol % n-Butan gefunden.

### Beispiel 9

In einem 100 ml-Autoklaven der Fa. Parr Instruments wurde bei 120 °C und 20 bar 6.8 g eines C-4-Gemisches mit folgender Zusammensetzung: 0.1 mol-% Isobutan, 27.6 mol-% n-Butan, 27.9 mol-% 1-Buten, 0.1 mol-% Isobuten und 44.0 mol-% 2-Butene hydroformyliert. Als Precursor wurden 0.0051 g Rh(acac)(CO)₂ in 42.29 g Toluol vorgelegt. Als Ligand wurden 0.0681 g der oben beschriebenen Ligandenmischung (³¹P-NMR: LI1a=91% + LI2a=9%) in der Katalysatoransatzlösung eingesetzt. Als organisches Amin wurden 0.0371 g der Verbindung (11) und 0.4960 g TIPB als GC-Standard zugefügt. Das Edukt wurde nach Erreichen der vorgesehenen Reaktionstemperatur zudosiert.

Während der Reaktion wurde der Druck über eine Synthesegasregelung mit Massedurchflussmesser konstant gehalten. Proben wurden aus der Reaktionsmischung nach 20 Stunden gezogen. Der Austrag enthält 60.45 mol-% n-Pentanal und 3.51 mol-% 2-Methylbutanal (Umsatz Butene 92.8 mol-%, Regioselektivität zu n-Pentanal 94.5 %). Als Hydrierprodukte werden im Austrag 0.1 mol-% Isobutan und 28.8 mol % n-Butan gefunden.

### Beispiel 10

In einem 100 ml-Autoklaven der Fa. Parr Instruments wurde bei 120 °C und 20 bar 6.8 g eines C-4-Gemisches mit folgender Zusammensetzung: 63.6 mol-% n-Butan, 1.0 mol-% 1-Buten und 35.8 mol % 2-Butene hydroformyliert. Als Precursor wurden 0.0049 g Rh(acac)(CO)₂ in 40.42 g Toluol vorgelegt. Als Ligand wurden 0.0651 g der oben beschriebenen Ligandenmischung (³¹P-NMR: LI1a=91% + LI2a=9%) in der Katalysatoransatzlösung eingesetzt. Als organisches Amin wurden 0.0354 g der Verbindung (11) und 0.4740 g TIPB als GC-Standard zugefügt. Das Edukt wurde nach Erreichen der vorgesehenen Reaktionstemperatur zudosiert.

Während der Reaktion wurde der Druck über eine Synthesegasregelung mit Massedurchflussmesser konstant gehalten. Proben wurden aus der Reaktionsmischung nach 20 Stunden gezogen. Der Austrag enthält 27.76 mol-% n-Pentanal und 2.14 mol-% 2-Methylbutanal (Umsatz Butene 81.0 mol-%, Regioselektivität zu n-Pentanal 92.8 %). Als Hydrierprodukte werden im Austrag 65.0 mol % n-Butan gefunden.

### Beispiel 11

In einem 100 ml-Autoklaven der Fa. Parr Instruments wurde bei 120 °C und 20 bar 6.8 trans-2-Buten hydroformyliert. Als Precursor wurden 0.0054 g Rh(acac)(CO)₂ in 43.78 g Toluol vorgelegt. Als Ligand wurden 0.0696 g der oben beschriebenen Ligandenmischung ³¹P-NMR: LI1a=91% + LI2a=9%) in der Katalysatoransatzlösung eingesetzt. Als organisches Amin wurden 0.0370 g der Verbindung (11) und 0.5121 g TIPB als GC-Standard zugefügt. Das Edukt wurde nach Erreichen der vorgesehenen Reaktionstemperatur zudosiert.

Während der Reaktion wurde der Druck über eine Synthesegasregelung mit Massedurchflussmesser konstant gehalten. Proben wurden aus der Reaktionsmischung nach 20 Stunden gezogen.

Der Austrag enthält 85.4 mol-% n-Pentanal und 5.95 mol-% 2-Methylbutanal (Regioselektivität zu n-Pentanal 93.4 %). Als Hydrierprodukte werden im Austrag 3.99 mol % n-Butan gefunden.

### Beispiel 12

In einem 100 ml-Autoklaven der Fa. Parr Instruments wurde bei 120 °C und 20 bar 6.0 g eines Kohlenwasserstoffgemisches aus katalytischen Spaltanlagen mit folgender Zusammensetzung: 1.5 mol-% Propan, 0.8 mol-% Propen, 28.1 mol% Isobutan, 8.1 mol-% n-Butan, 16.4 mol-% 1-Buten, 16.9 mol-% Isobuten, 28.2 mol-% 2-Butene, 0.5 mol-% 1,3-Butadien und Anteile an C5-Olefinen und -Kohlenwasserstoffen hydroformyliert. Als Precursor wurden 0.0046 g Rh(acac)(CO)₂ in 39.43 g Toluol vorgelegt. Als Ligand wurden 0.0672 g der oben beschriebenen Ligandenmischung ³¹P-NMR: LI1a=91% + LI2a=9%) in der Katalysatoransatzlösung eingesetzt. Als organisches Amin wurden 0.0331 g der Verbindung (11) und 0.4665 g TIPB als GC-Standard zugefügt. Das Edukt wurde nach Erreichen der vorgesehenen Reaktionstemperatur zudosiert. Während der Reaktion wurde der Druck über eine Synthesegasregelung mit Massedurchflussmesser konstant gehalten. Proben wurden aus der Reaktionsmischung nach 20 Stunden gezogen.

Der Austrag enthält 1.2 mol-% Propan, 0.68 mol% Butanal, 26.9 mol-% Isobutan, 9.66 mol-% n-Butan, 12.66 mol-% 3-Methylbutanal (74.8 % Isobutenumsatz), 39.5 mol-% Pentanal, 2.07 mol % 2-Methylbutanal (Umsatz n-Butene 97.9 %, Regioselektivtät zu n-Pentanal 95.0 %).

### Beispiel 13

In einem 100 ml-Autoklaven der Fa. Parr Instruments wurde bei 120 °C und 20 bar 5.8 g 1,3-Butadien hydroformyliert. Als Precursor wurden 0.0048 g Rh(acac)(CO)₂ in 41.19 g Toluol vorgelegt. Als Ligand wurden 0.0677 g der oben beschriebenen Ligandenmischung (³¹P-NMR: LI1a=91% + LI2a=9%) in der Katalysatoransatzlösung eingesetzt. Als organisches Amin wurden 0.0364 g der Verbindung (11) und 0.4991 g TIPB als GC-Standard zugefügt. Das Edukt wurde nach Erreichen der vorgesehenen Reaktionstemperatur zudosiert. Während der Reaktion wurde der Druck über eine Synthesegasregelung mit Massedurchflussmesser konstant gehalten. Proben wurden aus der Reaktionsmischung nach 20 Stunden gezogen.

Der Austrag enthält 0.26 mol-% n-Butan, 14.25 % n-Butene, 16.65 % Aldehyde und 9.68 mol-% 4-Vinyl-Cyclohexen. Der Gesamtumsatz an 1-3-Butadien beträgt 42.4 %.

### Beispiel 14

In einem 100 ml-Autoklaven der Fa. Parr Instruments wurde bei 120 °C und 50 bar 1.8 g Ethen hydroformyliert. Als Precursor wurden 0.0050 g Rh(acac)(CO)₂ in 42.68 g Toluol vorgelegt. Als Ligand wurden 0.0668 g der oben beschriebenen Ligandenmischung (³¹P-NMR: LI1a=91% + LI2a=9%) in der Katalysatoransatzlösung eingesetzt. Als organisches Amin wurden 0.0363 g der Verbindung (11) und 0.5095 g TIPB als GC-Standard zugefügt. Das Edukt wurde nach Erreichen der vorgesehenen Reaktionstemperatur zudosiert. Während der Reaktion wurde der Druck über eine Synthesegasregelung mit Massedurchflussmesser konstant gehalten. Proben wurden aus der Reaktionsmischung nach 20 Stunden gezogen. Der Umsatz zu Propanal beträgt 98.7 %.

### Beispiel 15

In einem 100 ml-Autoklaven der Fa. Parr Instruments wurde bei 120 °C und 20 bar 5.74 g Ölsäuremethylester hydroformyliert. Als Precursor wurden 0.0049 g Rh(acac)(CO)₂ in 42.00 g Toluol vorgelegt. Als Ligand wurden 0.0665 g der oben beschriebenen Ligandenmischung (³¹P-NMR: LI1a=91% + LI2a=9%) in der Katalysatoransatzlösung eingesetzt. Als organisches Amin wurden 0.0345 g der Verbindung (11) und 0.4956 g TIPB als GC-Standard zugefügt. Das Edukt wurde nach Erreichen der vorgesehenen Reaktionstemperatur zudosiert. Während der Reaktion wurde der Druck über eine Synthesegasregelung mit Massedurchflussmesser konstant gehalten. Proben wurden aus der Reaktionsmischung nach 20 Stunden gezogen. Aus ¹H- und ¹³C-NMR-Spektren wurden eine Aldehydausbeute von 43.3 mol-% berechnet. Die Regioselektivtät zu endständigen Aldeyden beträgt 22.2 mol-%. Der Doppelbindungsanteil beträgt 36.3 mol-%.

Für die nachfolgenden Versuche wurden die Liganden (3Ia) und (4IIa) sowie Kombinationen aus (3Ia) und (4I1a) untersucht.

### Beispiel 16 (Vergleichsbeispiel)

In einem 100 ml-Autoklaven der Fa. Parr Instruments wurde bei 120 °C und 20 bar 6,0 g cis-2-Buten hydroformyliert. Als Precursor wurden 0.0049 g Rh(acac)(CO)₂ in 44.38 g Toluol vorgelegt. Als Ligand wurden 0.0783 g Ligand (3Ia) in der Katalysatoransatzlösung eingesetzt. Als organisches Amin wurden 0.0392 g der Verbindung (11) und 0.4981 g TIPB als GC-Standard zugefügt. Das Edukt wurde nach Erreichen der vorgesehenen Reaktionstemperatur zudosiert.

Während der Reaktion wurde der Druck über eine Synthesegasregelung mit Massedurchflussmesser konstant gehalten. Proben wurden aus der Reaktionsmischung nach 12 Stunden gezogen. Es wurden 53.2 mol-% Pentanal, 16.6 mol-% 2-Methylbutanal und 3.19 mol-% n-Butan gebildet. Die Regioselektivität zu n-Pentanal beträgt 76.2 %.

### Beispiel 17 (Vergleichsbeispiel)

In einem 100 ml-Autoklaven der Fa. Parr Instruments wurde bei 120 °C und 20 bar 5.8 g cis-2-Buten hydroformyliert. Als Precursor wurden 0.006 g Rh(acac)(CO)₂ in 44.3 g Toluol vorgelegt. Als Ligand wurden 0.0907 g Ligand (4IIa) in der Katalysatoransatzlösung eingesetzt. Als organisches Amin wurden 0.0432 g der Verbindung (11) und 1.7624 g Mesitylen als GC-Standard zugefügt. Das Edukt wurde nach Erreichen der vorgesehenen Reaktionstemperatur zudosiert. Während der Reaktion wurde der Druck über eine Synthesegasregelung mit Massedurchflussmesser konstant gehalten. Proben wurden aus der Reaktionsmischung nach 12 Stunden gezogen. Es wurde eine Aldehydausbeute von 61.8 mol-% gefunden. Die Regioselektivtät zu n-Pentanal beträgt 76.2 mol-%. Der Anteil an n-Butan beträgt 3.2 %.

### Beispiel 18

In einem 100 ml-Autoklaven der Fa. Parr Instruments wurde bei 120 °C und 20 bar 6,4 g cis-2-Buten hydroformyliert. Als Precursor wurden 0.0047 g Rh(acac)(CO)₂ in 41.71 g Toluol vorgelegt. Als Liganden wurden 0.0674 g Ligand (3Ia) und 0.0075 g Ligand (4IIa) (molares Verhältnis L3Ia : L4IIa : Rh = 3.7: 0.41 : 1) in der Katalysatoransatzlösung eingesetzt. Als organisches Amin wurden 0.0346 g der Verbindung (11) und 1.8862 g Mesitylen als GC-Standard zugefügt. Das Edukt wurde nach Erreichen der vorgesehenen Reaktionstemperatur zudosiert.

Während der Reaktion wurde der Druck über eine Synthesegasregelung mit Massedurchflussmesser konstant gehalten. Proben wurden aus der Reaktionsmischung nach 12 Stunden gezogen. Es wurden 43.9 mol-% Pentanal, 13.0 mol-% 2-Methylbutanal und 2.66 mol-% n-Butan gebildet. Die Regioselektivität zu n-Pentanal beträgt 77.2 %.

### Beispiel 19

In einem 100 ml-Autoklaven der Fa. Parr Instruments wurde bei 120 °C und 20 bar 6,3 g cis-2-Buten hydroformyliert. Als Precursor wurden 0.0050 g Rh(acac)(CO)₂ in 41.17 g Toluol vorgelegt. Als Liganden wurden 0.0581 g Ligand (3Ia) und 0.0211 g Ligand (4IIa) (molares Verhältnis L3Ia : L4IIa : Rh = 2.96: 1.07 : 1) in der Katalysatoransatzlösung eingesetzt. Als organisches Amin wurden 0.0352 g der Verbindung (11) und 1.7344 g Mesitylen als GC-Standard zugefügt. Das Edukt wurde nach Erreichen der vorgesehenen Reaktionstemperatur zudosiert.

Während der Reaktion wurde der Druck über eine Synthesegasregelung mit Massedurchflussmesser konstant gehalten. Proben wurden aus der Reaktionsmischung nach 12 Stunden gezogen. Es wurden 46.0 mol-% Pentanal, 17.5 mol-% 2-Methylbutanal und 2.46 mol-% n-Butan gebildet. Die Regioselektivität zu n-Pentanal beträgt 72.4 %.

### Beispiel 20

In einem 100 ml-Autoklaven der Fa. Parr Instruments wurde bei 120 °C und 20 bar 6.2 g cis-2-Buten hydroformyliert. Als Precursor wurden 0.0055 g Rh(acac)(CO)₂ in 43.59 g Toluol vorgelegt. Als Ligand wurden 0.0389 g Ligand (3Ia) und 0.0388 g Ligand (4IIa) (molares Verhältnis L3Ia : L4IIa : Rh = 1.8 : 1.8 : 1) in der Katalysatoransatzlösung eingesetzt. Als organisches Amin wurden 0.0349 g der Verbindung (11) und 1.8283 g Mesitylen als GC-Standard zugefügt. Das Edukt wurde nach Erreichen der vorgesehenen Reaktionstemperatur zudosiert.

Während der Reaktion wurde der Druck über eine Synthesegasregelung mit Massedurchflussmesser konstant gehalten. Proben wurden aus der Reaktionsmischung nach 12 Stunden gezogen. Es wurden 42.8 mol-% Pentanal, 14.8 mol-% 2-Methylbutanal und 2.11 mol-% n-Butan gebildet. Die Regioselektivität zu n-Pentanal beträgt 74.4 %.

### Beispiel 21

In einem 100 ml-Autoklaven der Fa. Parr Instruments wurde bei 120 °C und 20 bar 6.3 g cis-2-Buten hydroformyliert. Als Precursor wurden 0.0047 g Rh(acac)(CO)₂ in 43.47 g Toluol vorgelegt. Als Ligand wurden 0.0203 g Ligand (3Ia) und 0.0576 g Ligand (4IIa) (molares Verhältnis L3Ia : L4IIa : Rh = 1.1 : 3.1 : 1) in der Katalysatoransatzlösung eingesetzt. Als organisches Amin wurden 0.0362 g der Verbindung (11) und 1.8681 g Mesitylen als GC-Standard zugefügt. Das Edukt wurde nach Erreichen der vorgesehenen Reaktionstemperatur zudosiert. Während der Reaktion wurde der Druck über eine Synthesegasregelung mit Massedurchflussmesser konstant gehalten. Proben wurden aus der Reaktionsmischung nach 12 Stunden gezogen. Es wurden 37.1 mol-% Pentanal, 14.3 mol-% 2-Methylbutanal und 1.52 mol-% n-Butan gebildet. Die Regioselektivität zu n-Pentanal beträgt 72.2 %.

### Beispiel 22

In einem 100 ml-Autoklaven der Fa. Parr Instruments wurde bei 120 °C und 20 bar 6.4 g cis-2-Buten hydroformyliert. Als Precursor wurden 0.0050 g Rh(acac)(CO)₂ in 43.06 g Toluol vorgelegt. Als Ligand wurden 0.0082 g Ligand (3Ia) und 0.0697 g Ligand (4IIa) (molares Verhältnis L3Ia : L4IIa : Rh = 0.41 : 3.55 : 1) in der Katalysatoransatzlösung eingesetzt. Als organisches Amin wurden 0.0374 g der Verbindung (11) und 1.7914 g Mesitylen als GC-Standard zugefügt. Das Edukt wurde nach Erreichen der vorgesehenen Reaktionstemperatur zudosiert.

Während der Reaktion wurde der Druck über eine Synthesegasregelung mit Massedurchflussmesser konstant gehalten. Proben wurden aus der Reaktionsmischung nach 12 Stunden gezogen. Es wurden 32.7 mol-% Pentanal, 12.5 mol-% 2-Methylbutanal und 1.12 mol-% n-Butan gebildet. Die Regioselektivität zu n-Pentanal beträgt 72.4 %.

### Beispiele für Langzeitversuche

### Beispiel L1: Hydroformylierung mit dem nicht erfindungsgemäßen Liganden (100) über 1200 h (Vergleichsbeispiel 1)

Der aus EP2280920B1 bekannte, nicht erfindungsgemäße Ligand der Formel (100) wurde in der Hydroformylierung einer Buten/Butan-Mischung eingesetzt.

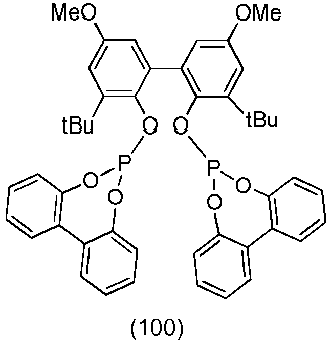

Dabei wurde Ligand (100) mit dem Amin der Formel (11) stabilisiert.

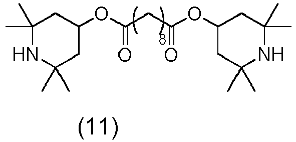

Die kontinuierlich betriebene Versuchsanlage bestand im Wesentlichen aus einem 20 Liter fassenden Druckreaktor mit einem nachgeschalteten Kondensator und Phasen-trennbehälter (Gas/Flüssigkeit) für die aus dem Reaktor stammende Gasphase sowie einem Kreisgasverdichter, der die Gasphase aus dem Phasentrennbehälter wieder unten in die Reaktionszone zurück führt. Ein Teil dieses Kreisgases wird nach der Phasentrennung als
Abgas aus dem Reaktionssystem gefahren. Um eine optimale Gasverteilung im Reaktorsystem zu realisieren, wurde hier ein Gasverteilerring mit Bohrungen verbaut. Über installierte Heizund Kühlvorrichtungen konnte der Reaktor temperiert werden.

Vor der Hydroformylierung wurde das System mit Stickstoff frei von Sauerstoff gespült. Anschließend wurde der Reaktor mit 12 Liter Katalysatorlösung gefüllt.

Diese Katalysatorlösung setzte sich aus 12 kg eines eutektischen Gemisches aus Biphenyl und Diphenylether (Diphyl® ,Wärmeträgeröl der Fa. Lanxess), 3 g Rh(acac)(CO)2, 36 g Bisphosphit-Ligand der Formel (100), 67.5 g Amin der Formel (11) zusammen und wurde vorher in einem Behälter gemischt. Das eutektische Gemisch aus Biphenyl und Diphenylether (Diphyl®) wurde zuvor mit Stickstoff gestrippt, um Sauerstoff und Wasser aus dem Wärmeträgeröl zu entfernen.

Anschließend wurde das Reaktorsystem mit Synthesegas frei von Stickstoff gespült. Nachdem der Stickstoffgehalt unter 10 Vol.-% gefallen war, wurde das Reaktorsystem mit Synthesegas auf 1.0 MPa aufgedrückt und anschließend auf 120 °C aufgeheizt. Nach Erreichen der Betriebstemperatur wurde das Reaktorsystem mit Synthesegas auf 1.7 MPa Reaktionsdruck gebracht.

Sodann wurde die Zugabe der Ausgangsstoffe gestartet. Hierzu wurde ein Einsatzgemisch über einen Verdampfer gefahren, um es gasförmig in das Kreisgas zu fahren. Bei dem Einsatzgemisch handelte es sich um eine Mischung aus 35 Gew.-% 2-Butenen und 1-Buten in einer Konzentration von ca. 1 %. Der Rest war n-Butan.

Folgende Durchsätze wurden eingestellt: 0,3 kg/h Einsatzgemisch, 75 Nl/h Synthesegas (50 Vol% H2 und 50 Vol% CO)
Zur täglichen Dosierung des Bisphosphit-Liganden (100) und Amins (11) wurde eine 1.4%-ige Lösung des Bisphosphit-Liganden (100) in n-Pentanal angesetzt, welches zuvor durch Strippen mit Stickstoff von restlichen C4-Kohlenwasserstoffen (< 3 %) befreit wurde. Das Amin (11) wurde in einem dreifachen molaren Überschuss zum Bisphosphit-Liganden (100) eingesetzt. Zur besseren Stabilisierung dieser Lösung wurde das Amin (11) vor dem Bisphosphit-Liganden (100) zur Lösung gegeben.

Nach ca. 1000 h wurde ein stationärer Zustand erreicht. Die Reaktionsprodukte wurden kontinuierlich über den Kreisgasstrom aus dem Reaktor entfernt und im Kondensator bei 50 °C partiell auskondensiert. Die auskondensierte Phase wurde kontinuierlich aus dem Phasentrennbehälter gefahren. Zur Umsatzbestimmung wurden aus dem Kreisgas vor und nach Reaktor Proben gezogen.

Durch eine tägliche Dosierung der oben beschriebenen Ligandenlösung, konnte der Umsatz und die Regioselektivität konstant gehalten werden.

Zur Bestimmung des Reaktorinhaltes wurden Proben aus dem Reaktor entnommen und mittels Flüssigchromatographie (HLPC) untersucht. Unter den gewählten Reaktionsbedingungen wurden Butenumsätze von rund 65 bis 70 % erzielt. Die prozentuale Verteilung zwischen n-Pentanal und 2-Methylbutanal, bzw. die n/iso-Selektivität, betrug 95 % zu 5 %.In der stationären Phase des Versuches konnte kein Rhodiumabbau verzeichnet werden.

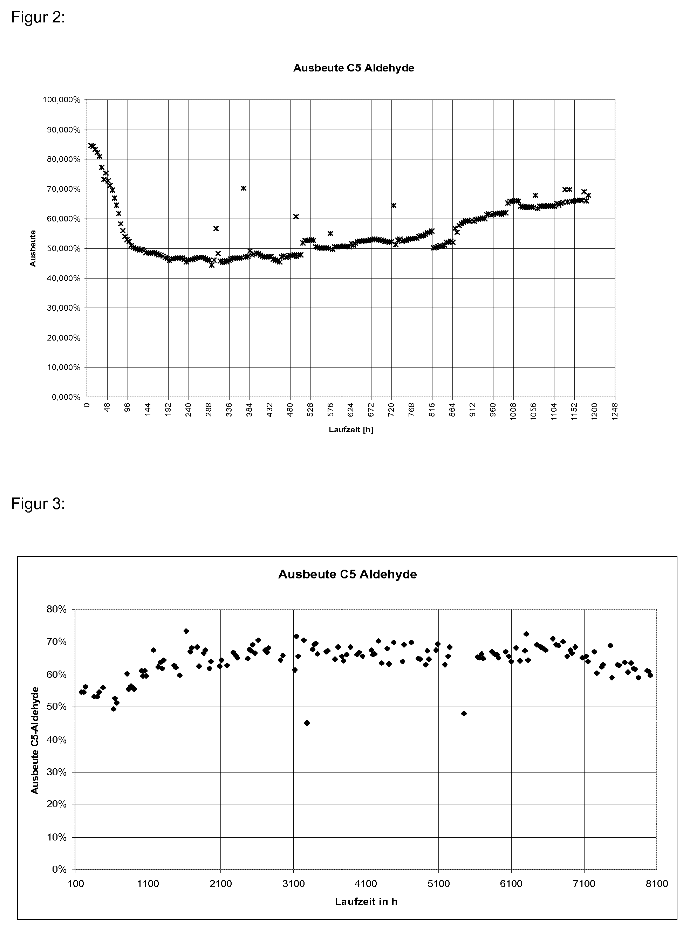

Die Ausbeute der C5-Aldehyde über die Versuchszeit ist in Figur 2 aufgetragen.

### Figur 2: Pentanal-Ausbeute zu Beispiel L1

Nach 1200 h wurde der Reaktor entspannt und die Katalysatorlösung untersucht. Im Reaktor zeigte sich ein Niederschlag. Eine Analyse dieses Niederschlages ergab, dass dieser aus phosphorhaltigen Folgeprodukten des Bisphosphit-Liganden (100) und dem eingesetzten Amin (11) bestanden. Es wurden keinerlei Anbackungen dieser Ausfällungen in dem Reaktor festgestellt.

Ein Teil des Reaktorinhaltes wurde, nach Abtrennung des Niederschlages, bei 1.2 KPa abs. und 220 °C Sumpftemperatur auf 13 % bezogen auf die Einsatzmasse eingeengt. Der erhaltene Rückstand aus dem Sumpf war noch fließfähig und es wurde kein Niederschlag festgestellt. Eine Rhodiumanalyse zeigte, dass das sich das gesamte Rhodium aus der Einsatzmasse in diesem Sumpfrückstand befand.

### Beispiel L2: Hydroformylierung mit dem nicht erfindungsgemäßen Liganden (100) über 8000 h (Vergleichsbeispiel 2)

Die Versuchsdurchführung fand in der, in Beispiel L1 beschriebenen Versuchsanlage statt. Die Vorbereitung des Versuches und die Durchführung fand analog zum Beispiel L1 statt.

In diesem Beispiel setzte sich die Katalysatorlösung aus 12 kg Isononylbenzoat, 4.5 g Rh(acac)(CO)2, 55 g Bisphosphit-Ligand der Formel (100), 67.5 g Amin der Formel (11) zusammen. Das Isononylbenzoat wurde ebenfalls zuvor mit Stickstoff gestrippt, um Sauerstoff und Wasser aus dem Lösemittel zu entfernen.

Anschließend wurde das Reaktorsystem mit Synthesegas frei von Stickstoff gespült. Nachdem der Stickstoffgehalt unter 10 Vol.-% gefallen war, wurde das Reaktorsystem mit Synthesegas auf 1.0 MPa aufgedrückt und anschließend auf 120 °C aufgeheizt. Nach Erreichen der Betriebstemperatur wurde das Reaktorsystem mit Synthesegas auf 1.7 MPa Reaktionsdruck gebracht.

Anschließend wurde die Zugabe der Ausgangsstoffe gestartet. Hierzu wurde ein Einsatzgemisch über einen Verdampfer gefahren, um es gasförmig in das Kreisgas zu fahren. Bei dem Einsatzgemisch handelte es sich um eine Mischung aus 35 Gew.-% 2-Butenen und 1-Buten in einer Konzentration von ca. 1 %. Der Rest war n-Butan. Folgende Durchsätze wurden eingestellt: 0,3 kg/h Einsatzgemisch, 75 Nl/h Synthesegas (50 Vol% H2 und 50 Vol% CO). Zur täglichen Dosierung des Bisphosphit-Liganden (100) und Amins (11) wurde eine 1.4%-ige Lösung des Bisphosphit-Liganden (100) in n-Pentanal angesetzt, welches zuvor durch Strippen mit Stickstoff von restlichen C4-Kohlenwasserstoffen (< 3 %) befreit wurde. Das Amin (11) wurde in einem dreifachen molaren Überschuss zum Bisphosphit-Liganden (100) eingesetzt. Zur besseren Stabilisierung dieser Lösung wurde das Amin (11) vor dem Bisphosphit-Liganden (100) zur Lösung gegeben.

Wie in Beispiel L1 wurde nach etwa 1000 h ein stationärer Zustand erreicht. Die Reaktionsprodukte wurden kontinuierlich über den Kreisgasstrom aus dem Reaktor entfernt und im Kondensator bei 50 °C partiell auskondensiert. Die auskondensierte Phase wurde kontinuierlich aus dem Phasentrennbehälter gefahren. Zur Umsatzbestimmung wurden aus dem Kreisgas vor und nach Reaktor Proben gezogen.

Durch eine tägliche Dosierung der oben beschriebenen Ligandenlösung, konnte der Umsatz und die Regioselektivität konstant gehalten werden.

Zur Bestimmung des Reaktorinhaltes wurden Proben aus dem Reaktor entnommen und mittels Flüssigchromatographie (HLPC) untersucht. Unter den gewählten Reaktionsbedingungen wurden Butenumsätze von rund 65 bis 70 % erzielt. Die prozentuale Verteilung zwischen n-Pentanal und 2-Methylbutanal, bzw. die n/iso-Selektivität, betrug 95 % zu 5 %.In der stationären Phase des Versuches konnte kein Rhodiumabbau verzeichnet werden.

Die Ausbeute der C5-Aldehyde über die Versuchszeit ist in Figur 3 aufgetragen.

### Figur 3: Pentanal-Ausbeute zu Beispiel L2

Nach 1500 h zeigten sich in den Proben aus dem Reaktor erste Niederschläge. Die Analyse dieser Niederschläge ergab, dass dieser, ebenso wie in Beispiel L1, aus phosphorhaltigen

Folgeprodukten des Bisphosphit-Liganden (100) und dem eingesetzten Amin (11) bestanden. Die Reaktion wurde insgesamt 8100 h betrieben, die Rhodiumverluste durch Probenahmen wurden durch Zugabe entsprechender Mengen Rh(acac)(CO)2 in die tägliche Ligandendosierungslösung ausgeglichen.

Im Verlauf wurde nach ca. 7000 h ein Aktivitätsrückgang in der Reaktion beobachtet und die Reaktionslösung neigte zum Schäumen. Der Prozess konnte nicht mehr betrieben werden und der Versuch musste beendet werden.

Nach Ende der Reaktion wurde der Reaktor entspannt und die Reaktionsmischung untersucht. Es zeigten sich große Mengen an Feststoff. 250 ml der Reaktionslösung wurden 4 h unter N2 Atmosphäre bei 40°C gerührt und anschließend die Viskosität des Rückstands vermessen. Die Viskosität betrug 300 mPas.

### Beispiel L3: Hydroformylierung mit erfindungsgemäßem Katalysatorsystem

Es wurde dieselbe Versuchsanlage eingesetzt wie in Beispiel L3. Es wurde dasselbe Einsatzgemisch und dasselbe Synthesegas verwendet. Als Ligand wurde indes eine Mischung aus den beiden Bisphosphit-Liganden (1Ia) und (2IIa) eingesetzt. Der aus EP2280920B1 bekannte Ligand der Formel (100) war nicht im Reaktionsgemisch enthalten. Es wurde
dasselbe Amin (11) wie im Vergleichsbeispiel 1 (L1) als Stabilisator verwendet. Als
Lösungsmittel wurde Isononylbenzoat eingesetzt.

Vor der Hydroformylierung wurde das System mit Stickstoff frei von Sauerstoff gespült. Anschließend wurde der Reaktor mit 12 Liter Katalysatorlösung gefüllt.

Diese Katalysatorlösung setzte sich aus 12 kg Isononylbenzoat, 4.5 g Rh(acac)(CO)2, 63 g Liganden-Isomerengemisch der Formeln (1Ia) und (2IIa), 200g Amin der Formel (11) zusammen und wurde vorher in einem Behälter gemischt. Das Isononylbenzoat wurde zuvor mit Stickstoff gestrippt, um Sauerstoff und Wasser aus dem Lösemittel zu entfernen. Anschließend wurde das Reaktorsystem mit Synthesegas frei von Stickstoff gespült. Nachdem der Stickstoffgehalt unter 10 Vol-% gefallen war, wurde das Reaktorsystem mit Synthesegas auf 1.0 MPa aufgedrückt und anschließend auf 120 °C aufgeheizt. Nach Erreichen der Betriebstemperatur wurde das Reaktorsystem mit Synthesegas auf 1.7 MPa Reaktionsdruck gebracht.

Sodann wurde die Zugabe der Ausgangsstoffe gestartet. Das Einsatzgemisch wurde über einen Verdampfer gefahren, um es gasförmig in das Kreisgas zu fahren. Folgende Durchsätze wurden eingestellt: 0.3 kg/h Einsatzgemisch, 75 Nl/h Synthesegas.

Zur täglichen Dosierung des Isomerengemisches bestehend aus (1Ia) und (2IIa) und Amins (11) wurde eine 1.4%-ige Lösung der Ligandenmischungen aus den Bisphosphit-Liganden (1Ia) und (2IIa) in n-Pentanal angesetzt, welches zuvor durch Strippen mit Stickstoff von restlichen C4-Kohlenwasserstoffen (< 3 %) befreit wurde. Das Amin (11) wurde in einem dreifachen molaren Überschuss zum Ligandenisomerengemisch bestehend aus (1Ia) und (2IIa) eingesetzt.

Zur besseren Stabilisierung dieser Lösung wurde das Amin (11) vor dem Bisphosphit-Ligandenisomerengemisch zur Lösung gegeben.

Die Reaktionsprodukte wurden kontinuierlich über den Kreisgasstrom aus dem Reaktor entfernt und im Kondensator bei 50 °C partiell auskondensiert. Die auskondensierte Phase wurde kontinuierlich aus dem Phasentrennbehälter gefahren. Zur Ausbeutebestimmung wurden aus dem Kreisgas vor und nach Reaktor Proben gezogen und mittels Gaschromatograph analysiert. Durch eine tägliche Dosierung der oben beschriebenen Ligandenlösung, konnte der Umsatz und die Regioselektivität konstant gehalten werden. Zur Bestimmung des Reaktorinhaltes wurden Proben aus dem Reaktor entnommen und mittels Flüssigchromatographie (HLPC)
untersucht.

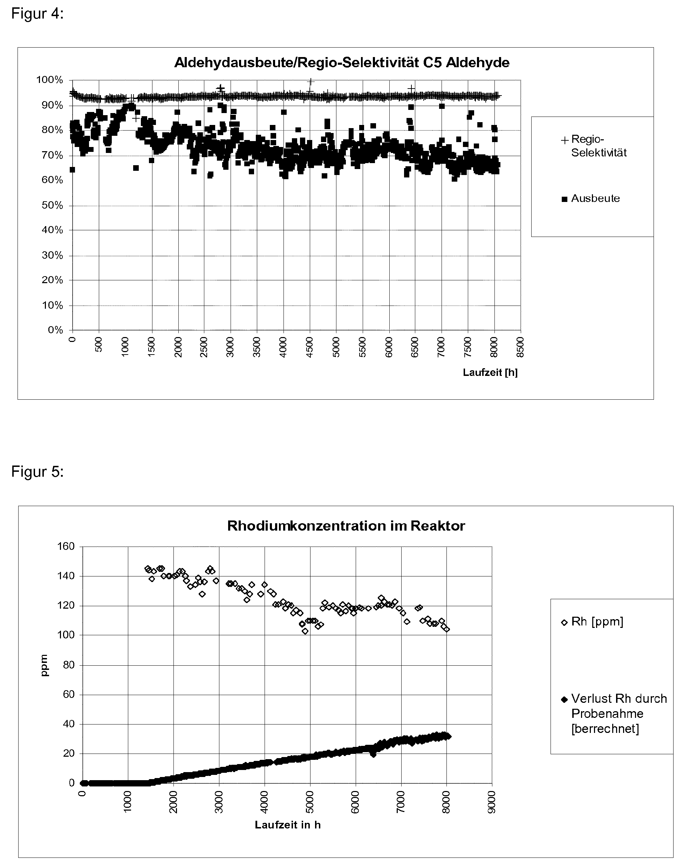

Unter den gewählten Reaktionsbedingungen stellte sich zum Start der Reaktion eine Aldehydausbeute zwischen 80% und 90% ein. Nach 8000 h Betriebszeit fiel die Ausbeute auf ca. 65% ab, bedingt durch die Rhodiumverluste durch die Probennahmen. Ein Schäumen der Reaktionslösung konnte in diesem Fall nicht festgestellt werden. Die prozentuale Verteilung zwischen n-Pentanal und 2-Methylbutanal, bzw. die Regio-Selektivität, betrug 92 % zu 8 %. Aldehydausbeute und Regio-Selektivität sind über die Versuchsdauer in Figur 4 aufgetragen.

### Figur 4: Aldehydausbeute und Regio-Selektivität zu Beispiel L3

In der stationären Phase des Versuches konnte, abgesehen von den Rhodiumverlusten durch die Probenahme, kein weiterer Rhodiumabbau verzeichnet werden.

Die Rhodium-Konzentration im Reaktor über die Versuchsdauer ist in Figur 5 aufgetragen.

### Figur 5: Rh-Konzentration zu Beispiel L3

Nach Ende der Reaktion wurde der Reaktor entspannt und die Reaktionsmischung untersucht. Es zeigte sich kein Feststoff. 250ml der Reaktionslösung wurden 4 h unter N2 Atmosphäre bei 40 °C gerührt und anschließend die Viskosität des Rückstands vermessen. Die Viskosität
betrug 20 mPas.

### Vergleich der Beispiele L1, L2 und L3

Vergleicht man die entsprechenden Beispiele, so hebt sich das erfindungsgemäß durchgeführte Beispiel L3 durch folgende Merkmale deutlich von den Beispielen L1 und L2 ab, die den Stand der Technik wiedergegeben:
Das erfindungsgemäße Beispiel L3 zeigt keine Einfahrphase, das heißt das System zeigt keinen Aktivitätsrückgang in den ersten 1000 h Betriebszeit und somit produziert die Anlage im erfindungsgemäßen Beispiel L3 im gleichen Zeitraum deutlich mehr Produkt.

Im Vergleichsbeispiel 2 (L2) fällt im Laufe der Reaktion Feststoff an, der nur über eine aufwendige Filtration entfernt werden kann. Das erfindungsgemäße Beispiel L3 zeigt auch nach über 8000 h keinen Feststoffanfall, somit kann in diesem Verfahren auf die Filtration verzichtet werden.

Das Vergleichsbeispiel 2 (L2) zeigt ein deutliches Schäumen der Reaktionslösung zum Ende des Versuches, sodass der Prozess nicht mehr betrieben werden kann. Ein solches Verhalten ließe sich nur durch aufwendige Schaumbrecher verhindern. Das erfindungsgemäße Verfahren
kommt ohne diese Hilfsmittel aus.

## Patentansprüche

1. Gemisch umfassend die Verbindungen (la) und (IIa): 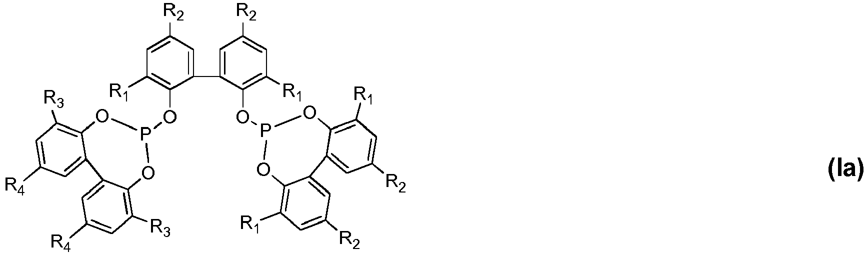 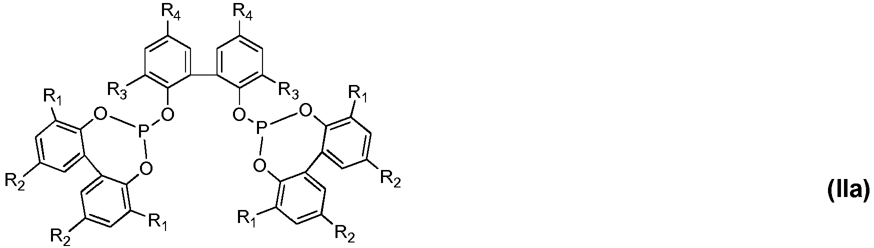 wobei
R1 ausgewählt ist aus -Me, -tBu, -OMe;
R2 ausgewählt ist aus -Me, -tBu, -OMe;
R3 ausgewählt ist aus -Me, -tBu, -OMe;
R4 ausgewählt ist aus -Me, -tBu, -OMe;
unter der Voraussetzung dass,
für den Fall dass R1 gleich R3 ist, R2 nicht gleich R4 ist,
für den Fall dass R2 gleich R4 ist, R1 nicht gleich R3 ist,
und P weitere Bindungen eingehen kann.

2. Gemisch nach Anspruch 1, wobei der Gehalt an Verbindung (la) in einem Bereich von 99,5 bis 0,5 Massen-%, der Gehalt an Verbindung (IIa) in einem Bereich von 0,5 bis 99,5 Massen-% liegt.

3. Gemisch nach einem der Ansprüche 1 oder 2, umfassend die Verbindungen (Ib) und (IIb): 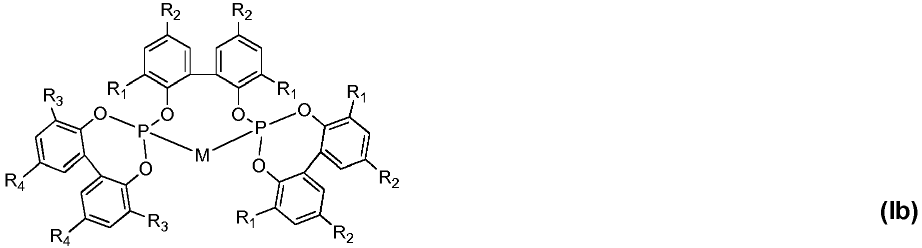 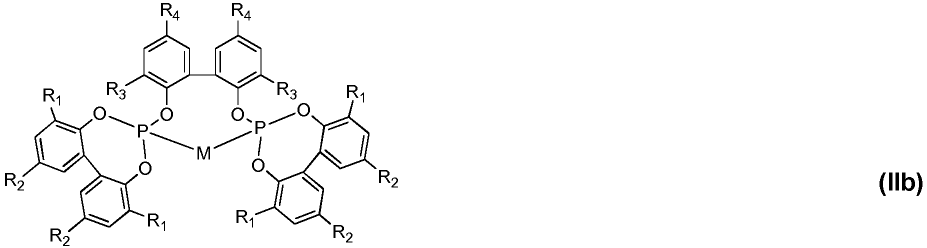 wobei M ausgewählt ist aus Fe, Ru, Os, Co, Rh, Ir, Ni, Pd, Pt,
und M zusätzliche Bindungen eingehen kann.

4. Gemisch nach einem der Ansprüche 1 bis 3,
umfassend die Verbindungen (Ic) und (IIc): 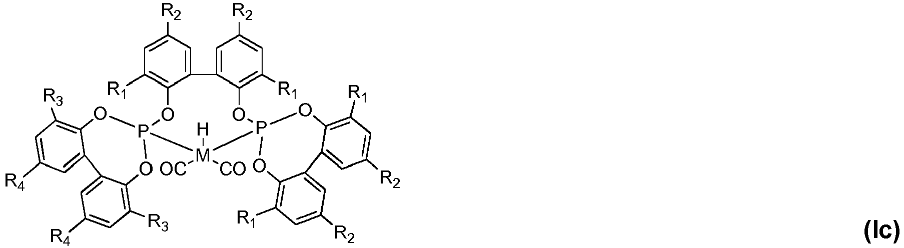 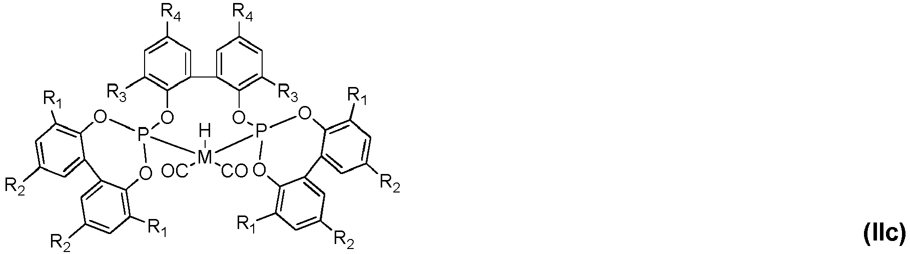 wobei M ausgewählt ist aus Fe, Ru, Os, Co, Rh, Ir, Ni, Pd, Pt.

5. Gemisch nach Anspruch 4,
welches zusätzlich mindestens eine Verbindung (la) oder (IIa) umfasst, die nicht an M gebunden ist.

6. Gemisch nach einem der Ansprüchen 4 oder 5
wobei M für Rh steht.

7. Gemisch nach einem der Ansprüche 1 bis 6,
wobei R1 -Me ist, und R3 nicht -Me ist.

8. Gemisch nach einem der Ansprüche 1 bis 7,
wobei R2 -Me ist, und R4 nicht -Me ist.

9. Gemisch nach einem der Ansprüche 1 bis 8,
wobei R1 und R2 -Me sind.

10. Gemisch nach einem der Ansprüche 1 bis 6,
wobei R1 -tBu ist, und R3 nicht -tBu ist.

11. Gemisch nach einem der Ansprüche 1 bis 6,
wobei R2 -OMe ist, und R4 nicht -OMe ist.

12. Zusammensetzung umfassend:
- ein Gemisch nach einem der Ansprüche 1 bis 11,
- eine weitere Komponente ausgewählt aus: Basen, organische Amine, Epoxide, Pufferlösungen, Ionenaustauscher.

13. Zusammensetzung nach Anspruch 12,
wobei das organische Amin zumindest eine 2,2,6,6-Tetramethylpiperidineinheit aufweist.

14. Verfahren zur Herstellung eines Gemisches nach Anspruch 1,
umfassend die Verfahrensschritte:
a) oxidative Kopplung gemäß Reaktionsschema A: 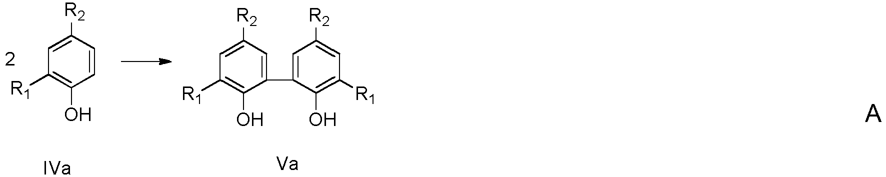
b) oxidative Kopplung gemäß Reaktionsschema B: 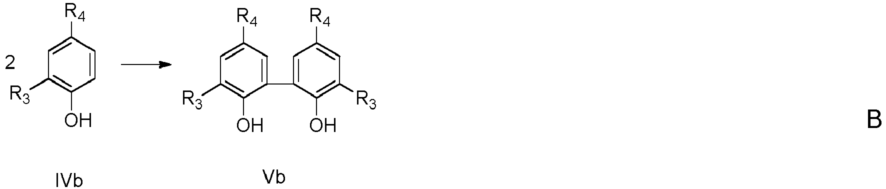
c) Umsetzung des Produktes aus a) mit PCl₃ gemäß Reaktionsschema C: 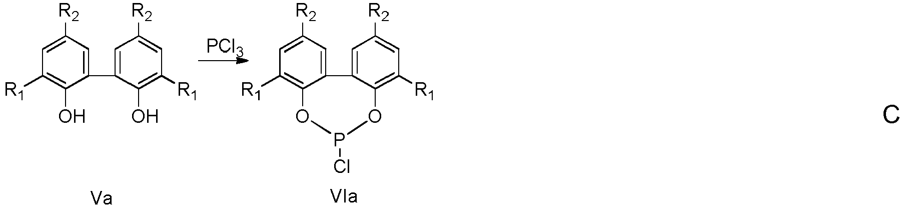
d) Umsetzung des Produktes aus b) mit dem Produkt aus c) zu einem Gemisch nach Anspruch 1.

15. Verfahren nach Anspruch 14,
zusätzlich umfassend den Verfahrenschritt:
e) Umsetzung mit M zu (Ib) und (IIb), wobei M ausgewählt ist aus Fe, Ru, Os, Co, Rh, Ir, Ni, Pd, Pt.

16. Verfahren zur Hydroformylierung von ungesättigten Verbindungen und deren Gemischen unter Verwendung:
- einer Zusammensetzung nach einem der Ansprüche 12 oder 13, und
- eines Gasgemisches umfassend Kohlenmonoxid und Wasserstoff.

17. Verfahren nach Anspruch 16, wobei die ungesättigten Verbindungen und deren Gemische ausgewählt sind aus:
- Kohlenwasserstoffgemischen aus Dampfspaltanlagen;
- Kohlenwasserstoffgemischen aus katalytisch betriebenen Spaltanlagen;
- Kohlenwasserstoffgemischen aus Oligomerisierungsprozessen;
- Kohlenwasserstoffgemischen umfassend mehrfach ungesättigte Verbindungen;
- ungesättigten Carbonsäurederivaten.

18. Verfahren nach Anspruch 17,
**dadurch gekennzeichnet, dass** die Kohlenwasserstoffgemische ungesättigte Verbindungen mit 2 bis 30 Kohlenstoffatomen aufweisen.

19. Verwendung eines Gemisches nach einem der Ansprüche 1 bis 11 als Katalysator in einer Hydroformylierungsreaktion von ungesättigten Verbindungen und deren Gemischen.

## Claims

1. Mixture comprising the compounds (Ia) and (IIa): 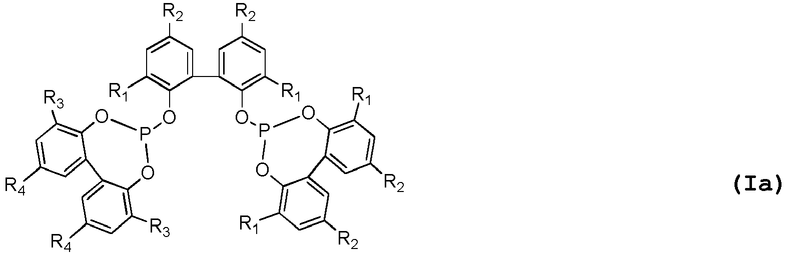 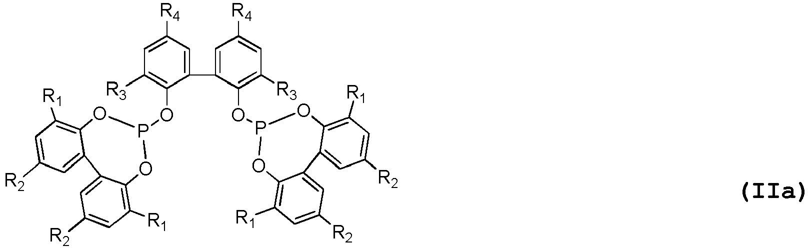 where
R1 is selected from -Me, -tBu, -OMe;
R2 is selected from -Me, -tBu, -OMe;
R3 is selected from -Me, -tBu, -OMe;
R4 is selected from -Me, -tBu, -OMe;
with the proviso that,
if R1 is the same as R3, R2 is not the same as R4,
if R2 is the same as R4, R1 is not the same as R3,
and P can enter into further bonds.

2. Mixture according to Claim 1, wherein the content of compound (Ia) is within a range from 99.5 to 0.5% by mass, and the content of compound (IIa) within a range from 0.5 to 99.5% by mass.

3. Mixture according to either of Claims 1 and 2, comprising the compounds (Ib) and (IIb): 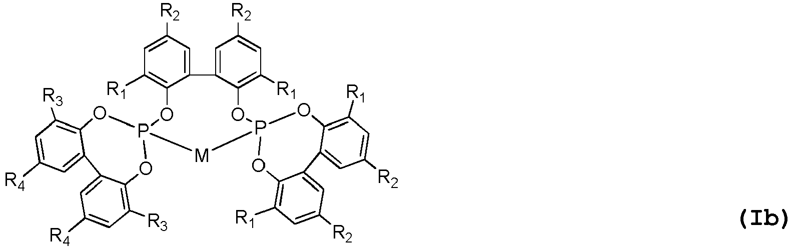 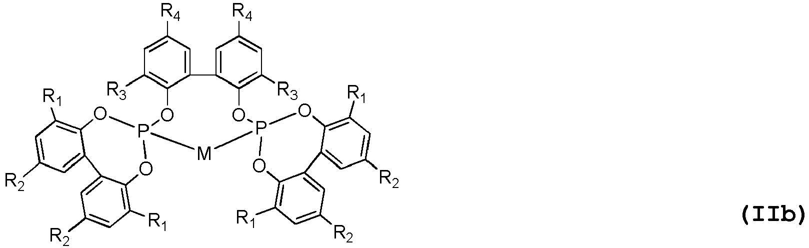 where M is selected from Fe, Ru, Os, Co, Rh, Ir, Ni, Pd, Pt,
and M can enter into additional bonds.

4. Mixture according to any of Claims 1 to 3, comprising the compounds (Ic) and (IIc): 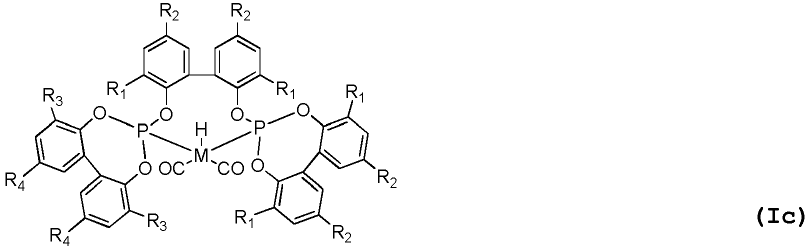 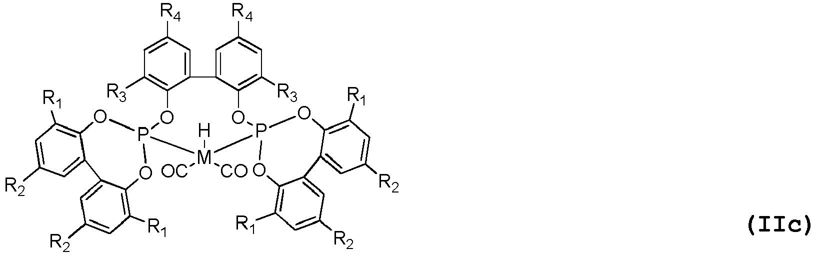 where M is selected from Fe, Ru, Os, Co, Rh, Ir, Ni, Pd, Pt.

5. Mixture according to Claim 4,
which additionally comprises at least one compound (Ia) or (IIa) not bonded to M.

6. Mixture according to either of Claims 4 and 5,
where M is Rh.

7. Mixture according to any of Claims 1 to 6,
where R1 is -Me, and R3 is not -Me.

8. Mixture according to any of Claims 1 to 7,
where R2 is -Me, and R4 is not -Me.

9. Mixture according to any of Claims 1 to 8,
where R1 and R2 are each -Me.

10. Mixture according to any of Claims 1 to 6,
where R1 is -tBu, and R3 is not -tBu.

11. Mixture according to any of Claims 1 to 6,
where R2 is -OMe, and R4 is not -OMe.

12. Composition comprising:
- a mixture according to any of Claims 1 to 11,
- a further component selected from: bases, organic amines, epoxides, buffer solutions, ion exchangers.

13. Composition according to Claim 12, wherein the organic amine has at least one 2,2,6,6-tetramethylpiperidine unit.

14. Process for preparing a mixture according to Claim 1,
comprising the process steps of:
a) oxidative coupling according to reaction scheme A: 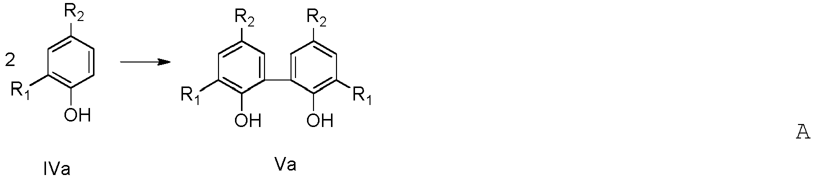
b) oxidative coupling according to reaction scheme B: 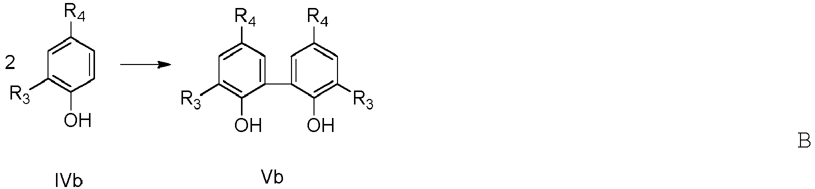
c) reaction of the product from a) with PCl₃ according to reaction scheme C: 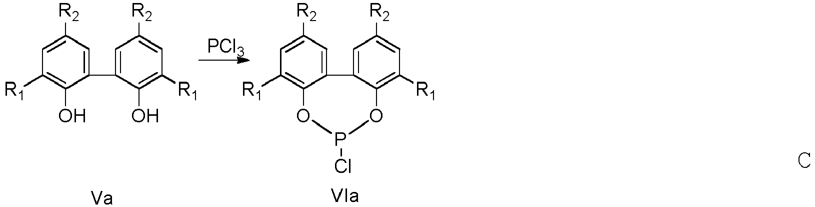
d) reaction of the product from b) with the product from c) to give a mixture according to Claim 1.

15. Process according to Claim 14,
additionally comprising the process step of:
e) reaction with M to give (Ib) and (IIb), where M is selected from Fe, Ru, Os, Co, Rh, Ir, Ni, Pd, Pt.

16. Process for hydroformylating unsaturated compounds and mixtures thereof using:
- a composition according to either of Claims 12 and 13, and
- a gas mixture comprising carbon monoxide and hydrogen.

17. Process according to Claim 16, wherein the unsaturated compounds and mixtures thereof are selected from:
- hydrocarbon mixtures from steamcracking plants;
- hydrocarbon mixtures from catalytically operated cracking plants;
- hydrocarbon mixtures from oligomerization operations;
- hydrocarbon mixtures comprising polyunsaturated compounds;
- unsaturated carboxylic acid derivatives.

18. Process according to Claim 17, **characterized in that** the hydrocarbon mixtures include unsaturated compounds having 2 to 30 carbon atoms.

19. Use of a mixture according to any of Claims 1 to 11 as a catalyst in a hydroformylation reaction of unsaturated compounds and mixtures thereof.

## Revendications

1. Mélange comprenant les composés (Ia) et (IIa) : 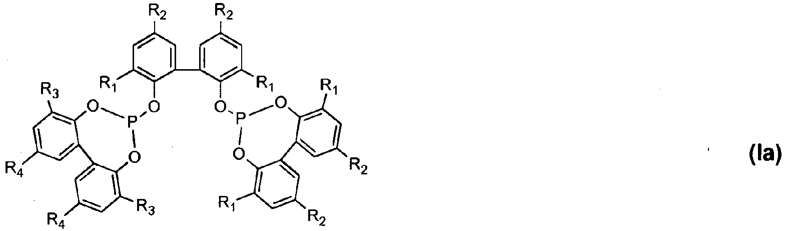 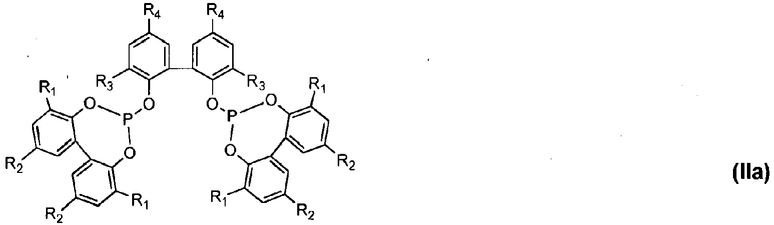 dans lesquels
R1 est choisi parmi -Me, -tBu, -OMe ;
R2 est choisi parmi -Me, -tBu, -OMe ;
R3 est choisi parmi -Me, -tBu, -OMe ;
R4 est choisi parmi -Me, -tBu, -OMe ;
à la condition que
dans le cas dans lequel R1 est égal à R3, R2 ne soit pas égal à R4,
dans le cas dans lequel R2 est égal à R4, R1 ne soit pas égal à R3,
et que P puisse établir des liaisons supplémentaires.

2. Mélange selon la revendication 1, dans lequel la teneur en le composé (Ia) est comprise dans la plage de 99,5 à 0,5 % en masse, la teneur en le composé (IIa) est comprise dans la plage de 0,5 à 99,5 % en masse.

3. Mélange selon l'une des revendications 1 ou 2, comprenant les composés (Ib) et (IIb) : 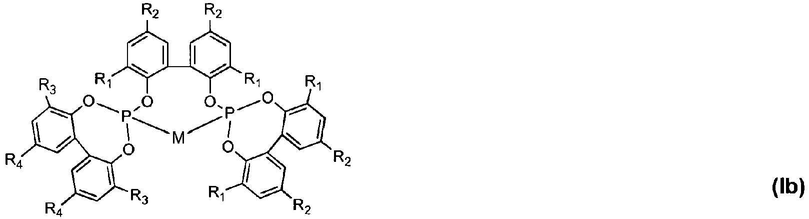 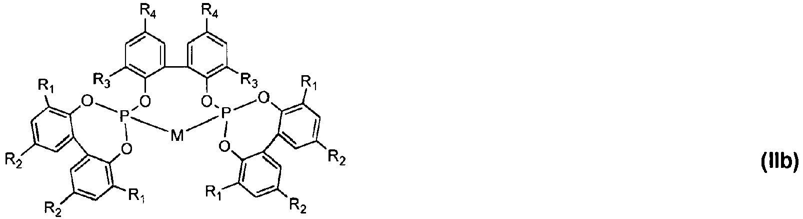 dans lesquels M est choisi parmi Fe, Ru, Os, Co, Rh, Ir, Ni, Pd, Pt,
et M peut établir des liaisons supplémentaires.

4. Mélange selon l'une des revendications 1 à 3, comprenant les composés (Ic) et (IIc) : 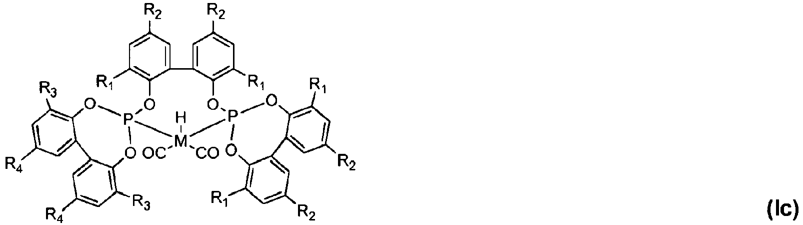 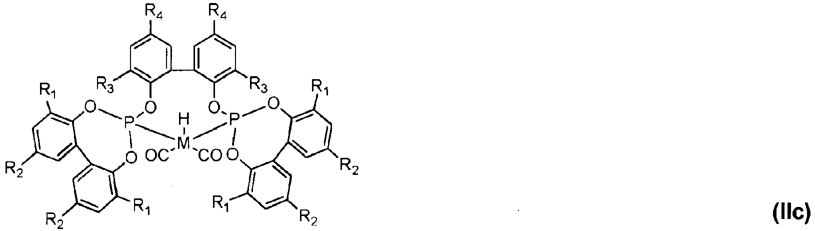 dans lesquels M est choisi parmi Fe, Ru, Os, Co, Rh, Ir, Ni, Pd, Pt.

5. Mélange selon la revendication 4, qui comprend en outre au moins un composé (Ia) ou (IIa) qui n'est pas lié à M.

6. Mélange selon l'une des revendications 4 ou 5, dans lequel M est Rh.

7. Mélange selon l'une des revendications 1 à 6, dans lequel R1 est -Me et R3 n'est pas -Me.

8. Mélange selon l'une des revendications 1 à 7, dans lequel R2 est -Me et R4 n'est pas -Me.

9. Mélange selon l'une des revendications 1 à 8, dans lequel R1 et R2 sont -Me.

10. Mélange selon l'une des revendications 1 à 6, dans lequel R1 est -tBu et R3 n'est pas -tBu.

11. Mélange selon l'une des revendications 1 à 6, dans lequel R2 est -OMe et R4 n'est pas -OMe.

12. Composition comprenant :
- un mélange selon l'une des revendications 1 à 11,
- un composant supplémentaire choisi parmi les bases, les amines organiques, les époxydes, les solutions tampon, les échangeurs d'ions.

13. Composition selon la revendication 12, dans laquelle l'amine organique comprend au moins un motif 2,2,6,6-tétraméthylpipéridine.

14. Procédé de préparation d'un mélange selon la revendication 1, comprenant les étapes suivantes :
a) couplage oxydatif selon le schéma réactionnel A : 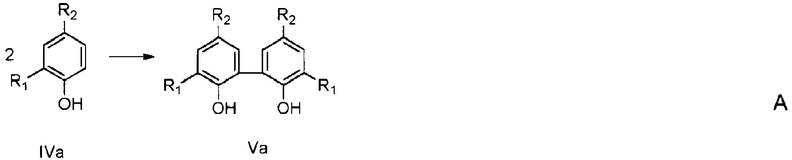
b) couplage oxydatif selon le schéma réactionnel B : 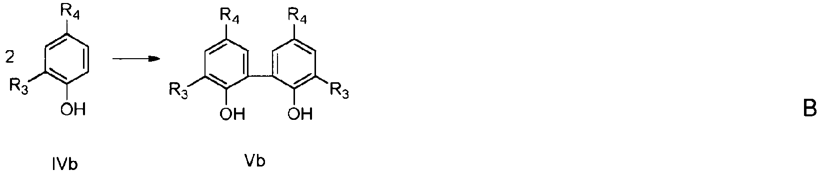
c) réaction du produit de a) avec du PCl₃ selon le schéma réactionnel C : 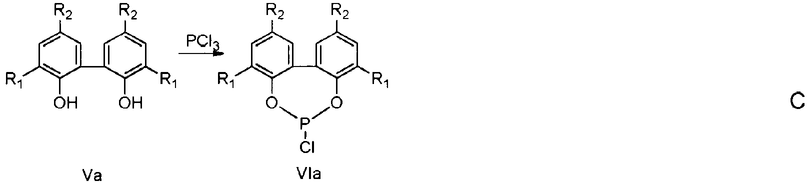
d) réaction du produit de b) avec le produit de c) pour donner un mélange selon la revendication 1.

15. Procédé selon la revendication 14, comprenant en outre l'étape suivante :
e) réaction avec M pour donner (Ib) et (IIb), M étant choisi parmi Fe, Ru, Os, Co, Rh, Ir, Ni, Pd, Pt.

16. Procédé pour l'hydroformylation de composés insaturés et de leurs mélanges, par utilisation :
- d'une composition selon l'une des revendications 12 ou 13, et
- d'un mélange gazeux comprenant du monoxyde de carbone et de l'hydrogène.

17. Procédé selon la revendication 16, dans lequel les composés insaturés et leurs mélanges sont choisis parmi :
- les mélanges d'hydrocarbures provenant d'installations de vapocraquage ;
- les mélanges d'hydrocarbures provenant d'installations de craquage catalytique ;
- les mélanges d'hydrocarbures provenant de procédés d'oligomérisation ;
- les mélanges d'hydrocarbures comprenant des composés polyinsaturés ;
- les dérivés d'acides carboxyliques insaturés.

18. Procédé selon la revendication 17, **caractérisé en ce que** les mélanges d'hydrocarbures comprennent des composés insaturés ayant 2 à 30 atomes de carbone.

19. Utilisation d'un mélange selon l'une des revendications 1 à 11 en tant que catalyseur dans une réaction d'hydroformylation de composés insaturés et de leurs mélanges.
